(19) Europäisches Patentamt
European Patent Office
Office européen des brevets

(11) Publication number: 0 139 515
A2

# (12) EUROPEAN PATENT APPLICATION

(21) Application number: 84306984.0

(22) Date of filing: 12.10.84

(51) Int. Cl.[4]: C 07 C 177/00
C 07 C 147/00, C 07 C 149/00
A 61 K 31/557

(30) Priority: 26.10.83 JP 201834/83

(43) Date of publication of application:
02.05.85 Bulletin 85/18

(84) Designated Contracting States:
AT BE CH DE FR GB IT LI NL SE

(71) Applicant: SUMITOMO CHEMICAL COMPANY, LIMITED
15 Kitahama 5-chome Higashi-ku
Osaka-shi Osaka 541(JP)

(72) Inventor: Sugie, Akihiro
10-1-116, Sonehigashinocho-2-chome
Toyonaka-shi(JP)

(72) Inventor: Muraoka, Masami
54-1-1011, Mukaigaoka-1-chome
Toyonaka-shi(JP)

(72) Inventor: Nakamura, Toshio
8-3-104, Hamakosien-3-chome
Nishinomiya-shi(JP)

(72) Inventor: Ono, Keiichi
5-3-530, Higashiawajicho-1-chome
Higashiyodogawa-ku Osaka(JP)

(72) Inventor: Yamamoto, Michihiro
16-40-309, Takagihigashimachi
Nishinomiya-shi(JP)

(74) Representative: Paget, Hugh Charles Edward et al,
MEWBURN ELLIS & CO. 2/3 Cursitor Street
London EC4A 1BQ(GB)

## (54) Novel bicyclooctane compounds and production thereof.

(57) A compound which has strong anti-ulcer activity and is useful in the treatment of ulcers has the formula:

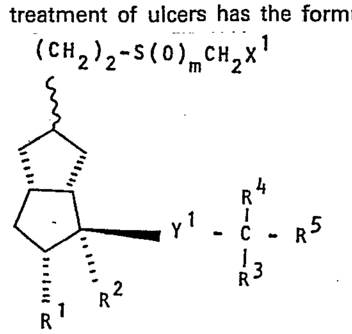

wherein $X^1$ is a free or esterified carboxyl group, or a group of the formula:

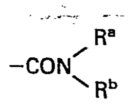

($R^a$ and $R^b$ are each independently a hydrogen atom, a $C_1$-$C_4$ alkyl group, a $C_3$-$C_7$ cycloalkyl group, a benzyl group, a phenyl group, a phenyl group substituted with a halogen atom or a $C_1$-$C_4$ alkyl group, or, when taken together with the adjacent nitrogen atom to which they are attached, they represent a 5 to 7 membered saturated heterocyclic group), $Y^1$ is a group of the formula:

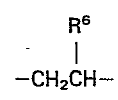

($R^6$ is a hydrogen atom or $C_1$-$C_4$ alkyl group), or

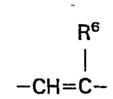

($R^6$ is as defined above),
$R^1$ is a hydrogen atom, a hydroxyl group or a protected hydroxyl group, $R^2$ is a hydrogen atom or $R^1$ and $R^2$, when taken together, mean a single linkage to form a double bond between the carbon atoms to which they are linked, $R^3$ is a hydroxyl group or a protected hydroxyl group, $R^4$ is a hydrogen atom or a $C_1$-$C_4$ alkyl group, $R^5$ is a hydrogen atom, a $C_1$-$C_{12}$ alkyl group, a $C_2$-$C_{12}$ alkenyl group, a $C_2$-$C_{12}$ alkynyl group, a $C_3$-$C_{10}$ cycloalkyl group, a $C_4$-$C_{10}$ cycloalkenyl group, a hydroxy $C_1$-$C_{12}$ alkyl group, a $C_3$-$C_{10}$ heterocyclic group, a

./...

EP 0 139 515 A2

phenyl group optionally substituted with a halogen atom, a hydroxyl group, a $C_1$-$C_4$ alkyl group a trifluoromethyl group, or a $C_1$-$C_4$ alkyl group or a group of the formula. A-B (A is a $C_1$-$C_7$ alkylene chain and B is a $C_3$-$C_{10}$ cycloalkyl group, a $C_4$-$C_{10}$ cycloalkenyl group, a $C_1$-$C_{12}$ alkoxy group, a $C_3$-$C_{10}$ cycloalkoxy group, a $C_4$-$C_{10}$ cycloalkenyloxy group, a $C_3$-$C_{10}$ heterocyclic group, or a phenyl or phenoxy group optionally substituted with a halogen atom, a hydroxy group, a $C_1$-$C_4$ alkyl group. a trifluoromethyl group or a $C_1$-$C_4$ alkoxy group) m is 0, 1 or 2; or a non-toxic pharmaceutically acceptable salt thereof.

# NOVEL BICYCLOOCTANE COMPOUNDS AND PRODUCTION THEREOF

The present invention relates to novel bicyclooctane compounds, their production and use.

More particularly, this invention relates to novel bicyclooctane compounds, to a pharmaceutical composition containing at least one of the bicyclooctane compounds and to a process for production thereof.

The novel bicyclooctane compounds provided by the present invention are those represented by the formula [I]:

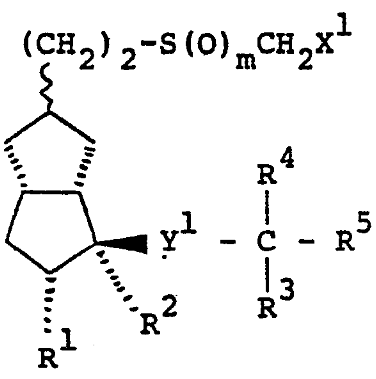

[I]

wherein $X^1$ is a free or esterified carboxyl group, or a group of the formula:

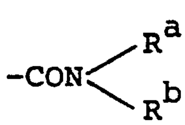

($R^a$ and $R^b$ are each independently a hydrogen atom, a $C_1$-$C_4$ alkyl group, a $C_3$-$C_7$ cycloalkyl group, a benzyl group, a phenyl group, a phenyl group substituted with a halogen

atom or a $C_1$-$C_4$ alkyl group, or, when taken together with the adjacent nitrogen atom to which they are attached, they represent a 5 to 7 membered saturated heterocyclic group), $Y^1$ is a group of the formula:

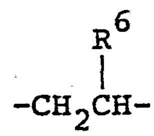

($R^6$ is a hydrogen atom or $C_1$-$C_4$ alkyl group), or

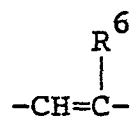

($R^6$ is as defined above),

$R^1$ is a hydrogen atom, a hydroxyl group or a protected hydroxyl group, $R^2$ is a hydrogen atom or $R^1$ and $R^2$, when taken together, mean a single linkage to form a double bond between the carbon atoms which they are linked, $R^3$ is a hydroxyl group or a protected hydroxyl group, $R^4$ is a hydrogen atom or a $C_1$-$C_4$ alkyl group, $R^5$ is a hydrogen atom, a $C_1$-$C_{12}$ alkyl group, a $C_2$-$C_{12}$ alkenyl group, a $C_2$-$C_{12}$ alkynyl group, a $C_3$-$C_{10}$ cycloalkyl group, a $C_4$-$C_{10}$ cycloalkenyl group, a hydroxy $C_1$-$C_{12}$ alkyl group, a $C_3$-$C_{10}$ heterocyclic group, a phenyl group optionally substituted with a halogen atom, a hydroxyl group, a $C_1$-$C_4$ alkyl group, a trifluoromethyl group or a $C_1$-$C_4$ alkoxy group, or a group of the formula: A-B (A is a $C_1$-$C_7$ alkylene chain and B is a $C_3$-$C_{10}$ cycloalkyl group, a $C_4$-$C_{10}$ cycloalkenyl group, a $C_1$-$C_{12}$ alkoxy group, a $C_3$-$C_{10}$ cycloalkoxy group, a $C_4$-$C_{10}$ cycloalkenyloxy

group, a $C_3$-$C_{10}$ heterocyclic group, or a phenyl or phenoxy group optionally substituted with a halogen atom, a hydroxy group, a $C_1$-$C_4$ alkyl group, a trifluoromethyl group or a $C_1$-$C_4$ alkoxy group), and m is 0, 1 or 2.

In the significances as used above, the term "halogen" includes fluorine, chlorine, bromine and iodine; the terms "$C_1$-$C_4$ alkyl" and "$C_1$-$C_4$ alkoxy" each means straight or branched chain alkyl and alkoxy group having from 1 to 4 carbon atoms (e.g. methyl, ethyl, n-propyl, isopropyl, n-butyl, methoxy, ethoxy, n-propoxy, isopropoxy, n-butoxy, etc.).

The term "$C_1$-$C_{12}$ alkyl" in the both cases, "$C_1$-$C_{12}$ alkyl" and "$C_1$-$C_{12}$ alkyl in the $C_1$-$C_{12}$ alkoxy group" means a straight or branched chain alkyl group having from one to 12 carbon atoms (e.g. methyl, ethyl, n-propyl, isopropyl, n-butyl, isobutyl, n-pentyl, isopentyl, 1-methylpentyl, 2-methylpentyl, 1,1-dimethylpentyl, 1-ethylpentyl, 2-ethylpentyl, n-hexyl, 1-methylhexyl, 2-methylhexyl, 3-methylhexyl, heptyl, 1-methylheptyl, 2-methylheptyl, 1-ethylheptyl, 2-ethylheptyl, n-octyl, 1-methyloctyl, 2-methyloctyl, 1-ethyloctyl, 2-ethyloctyl, 2,6-dimethylheptyl, 1,6-dimethylheptyl, n-nonyl, 1-methylnonyl, 2-methylnonyl, n-decyl, 1-methyldecyl, 2-methyldecyl, 2-ethyldecyl etc.).

The terms "$C_2$-$C_{12}$ alkenyl" and "$C_2$-$C_{12}$ alkynyl" mean straight or branched chain alkenyl group or alkynyl group having from 2 to 12 carbon atoms (e.g. vinyl, 2-propenyl, 2-butenyl, 2-pentenyl, 2-hexenyl,

5-heptenyl, 6-methyl-5-heptenyl, 2,6-dimethyl-5-heptenyl, 3-pentenyl, 4-pentenyl, 2,6-dimethyl-5-octenyl, 1,1,6-trimethyl-5-heptenyl, 4,8-dimethyl-7-nonenyl, 2,6-dimethyl-1,5-heptadienyl, 2-propynyl, 1-methylenepentyl, 2-butynyl, 2-pentynyl, 3-pentynyl, 1-methyl-3-pentynyl, 4-pentynyl, 4-hexynyl, 5-heptynyl, 6-heptynyl, 2-methyl-5-heptynyl, etc.).

The term "$C_3$-$C_{10}$ cycloalkyl" in the both cases, "$C_3$-$C_{10}$ cycloalkyl" and "$C_3$-$C_{10}$ cycloalkyl in the $C_3$-$C_{10}$ cycloalkoxy group" means cyclic alkyl group which has from 3 to 10 carbon atoms and which is optionally substituted with $C_1$-$C_4$ alkyl group or alkenyl group (e.g. cyclopropyl, cyclobutyl, cyclopentyl, cyclohexyl, cycloheptyl, 2-isopropylidenemethyl-3,3-dimethylcyclopropyl, 2-propylcyclopropyl, 3-ethylcyclobutyl, 3-ethylcyclopentyl, 4-methylcyclohexyl, 3-ethylcyclohexyl, 4-methylcycloheptyl, 2-isopropyl-5-methylcyclohexyl, norbornyl, adamantyl etc.). The term "alkenyl" means straight or branched chain alkenyl group having from 1 to 4 carbon atoms (e.g. vinyl, allyl, 1-propenyl, isopropenyl, 3-butenyl, 2-butenyl, isobutenyl, 1-isobutenyl).

The term "$C_4$-$C_{10}$ cycloalkenyl" in the both cases, "$C_4$-$C_{10}$ cycloalkenyl" and "$C_4$-$C_{10}$ cycloalkenyl in the $C_4$-$C_{10}$ cycloalkenyloxy" means cyclic alkenyl group having from 4 to 10 carbon atoms (e.g. bicyclo[4,3,0]nona-3-en-8-yl, 3-cyclopentenyl, 3-cyclohexenyl, 3-cycloheptenyl, tetrahydro-2-indanyl etc.).

The term "hydroxy $C_1$-$C_{12}$ alkyl" means straight or branched alkyl group which has from one to 12 carbon atoms and is substituted with hydroxyl group (e.g. hydroxymethyl, 2-hydroxyethyl, 3-hydroxypropyl, 4-hydroxybutyl, 5-hydroxypentyl, 6-hydroxyhexyl, 7-hydroxyheptyl, 8-hydroxyoctyl, 10-hydroxydecyl, 5-hydroxyhexyl, 4-hydroxypentyl, 5-hydroxyl-1,1-dimethylpentyl, 5-hydroxy-2-methylpentyl, 5-hydroxy-1-methylpentyl, 6-hydroxy-2-methylhexyl etc.).

The term "$C_3$-$C_{10}$ heterocyclic group" means mono or dicyclic group having from 3 to 10 carbon atoms and at least one of hetero atoms selected from nitrogen atoms, sulfur atoms, and oxygen atoms (e.g. piperidine, morpholine, pyrrolidine, piperazine, tetrahydrofuran, tetrahydrothiophene, furan, thiophene, imidazole, pyridine, oxazole, isooxazole, pyrrole, pyrazole, pyrimidine, indole, benzofuran, purine, benzothiophene, quinoline, pyrrolidone, dihydrothiophene, dihydrobenzofuran, 1,4-benzodioxane, etc.).

The term "$C_1$-$C_7$ alkylene" means straight or branched alkylene chain having from one to 7 carbon atoms (e.g. methylene, ethylene, trimethylene, tetramethylene, pentamethylene, hexamethylene, heptamethylene, methylmethylene, dimethylmethylene, 1,1-dimethylethylene, 2-methyltetramethylene, 1-methylpentamethylene, 2-methylhexamethylene, 1-ethylethylene, 2-ethylethylene, 2-ethyltrimethylene, etc.).

The term "5 to 7 membered saturated heterocyclic

group" includes piperidine, morpholine, pyrrolidine, homopiperidine, piperazine, N-($C_1$-$C_4$) alkylpiperazine, etc.).

The term "$C_3$-$C_7$ cycloalkyl" means a cyclic alkyl group which has from 3 to 7 carbon atoms and which is optionally substituted with a $C_1$-$C_4$ alkyl group (e.g. cyclopropyl, cyclobutyl, cyclopentyl, cyclohexyl, cycloheptyl, 3-ethylcyclopentyl, 4-methylcyclohexyl, etc.).

The term "esterified carboxyl group" includes $C_1$-$C_4$ alkoxycarbonyl, aryloxycarbonyl (e.g. phenoxycarbonyl, naphthoxycarbonyl), aralkyloxycarbonyl (e.g. benzyloxycarbonyl, phenethyloxycarbonyl), ($C_1$-$C_4$ alkoxy)methoxycarbonyl, ($C_2$-$C_5$ alkanoyloxy)methoxycarbonyl (e.g. acetoxymethoxycarbonyl), ($C_3$-$C_7$ cycloalkyloxy)carbonyl, arylcarbonylmethoxycarbonyl and (hydroxy $C_1$-$C_4$ alkoxy)carbonyl.

The term "protected hydroxy group" includes a hydroxy group protected with $C_1$-$C_4$ alkanoyl (e.g. acetyl, propionyl), benzoyl, substituted benzoyl, tetrahydropyranyl, tetrahydrofuryl or ($C_1$-$C_4$ alkoxy)methyl.

A tremendous amount of research in synthetic organic chemistry, pharmacology and clinical medicine of prostaglandins has been performed since discovery of prostaglandins.

In 1976, J. Vane of Wellcome foundation reported isolation and biological effects of prostacyclin [prostaglandin $I_2$ (referred to as "$PGI_2$", hereinafter)]. [S. Moncada, R. Gryglewski, S. Bunting, and J.R. Vane, Nature (London), 263, 663 (1976)].

$PGI_2$ [II], which is shown below, has several

excellent pharmacological activities, for example, hypotensive, vasodilating, antiallergic, antiulcerogenic, antithrombotic, anti cancerous activity, and is expected to be useful in treating asthma, ulcer, thrombosis, hypertention or cancer.

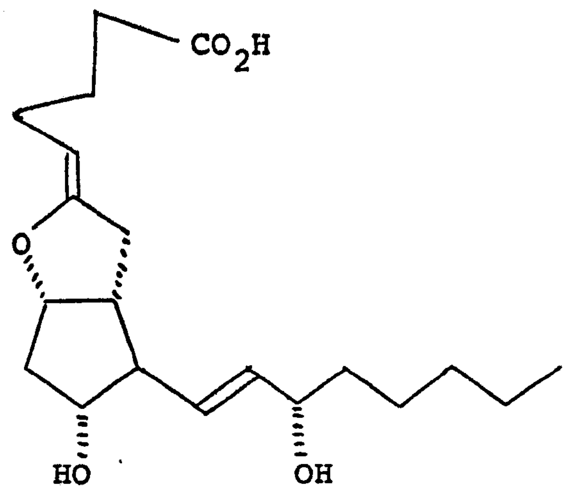

[II]

However, $PGI_2$ may not be used as medicine owing to its instability.

As the result of a study, it has now been found that the novel bicyclooctane compounds [I] of the present invention and their non-toxic pharmaceutically acceptable salts have selective anti-ulcerous action and are useful as anti-ulcerous drugs. In addition, the undesirable instability is absent in the compounds [I] of this invention.

Accordingly, a basic object of the present invention is to provide novel and stable bicyclooctane compounds [I] having excellent pharmacological activity.

Another object of the present invention is to provide a process for producing those compounds [I].

Further object of the present invention is to provide a pharmaceutical composition containing a compound of the formula [I]. These and other objects will be apparent to those skilled in the art to which the present invention pertains from the foregoing and subsequent descriptions.

The novel bicyclooctane compound [I] of the invention can be prepared by the following three methods.

A) The bicyclooctane compound of the formula, [I]

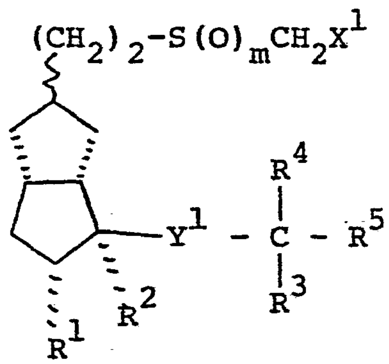

[I]

wherein $X^1$, $Y^1$, $R^1$, $R^2$, $R^3$, $R^4$, $R^5$ and m are each as defined above, can be prepared from an olefinic compound of the formula [III] by the process shown in the Scheme-A:

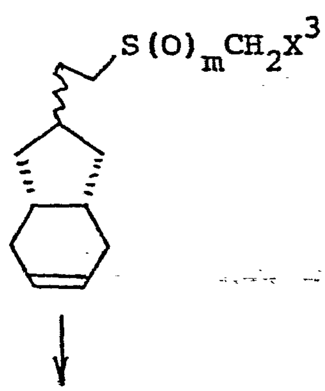

[III]

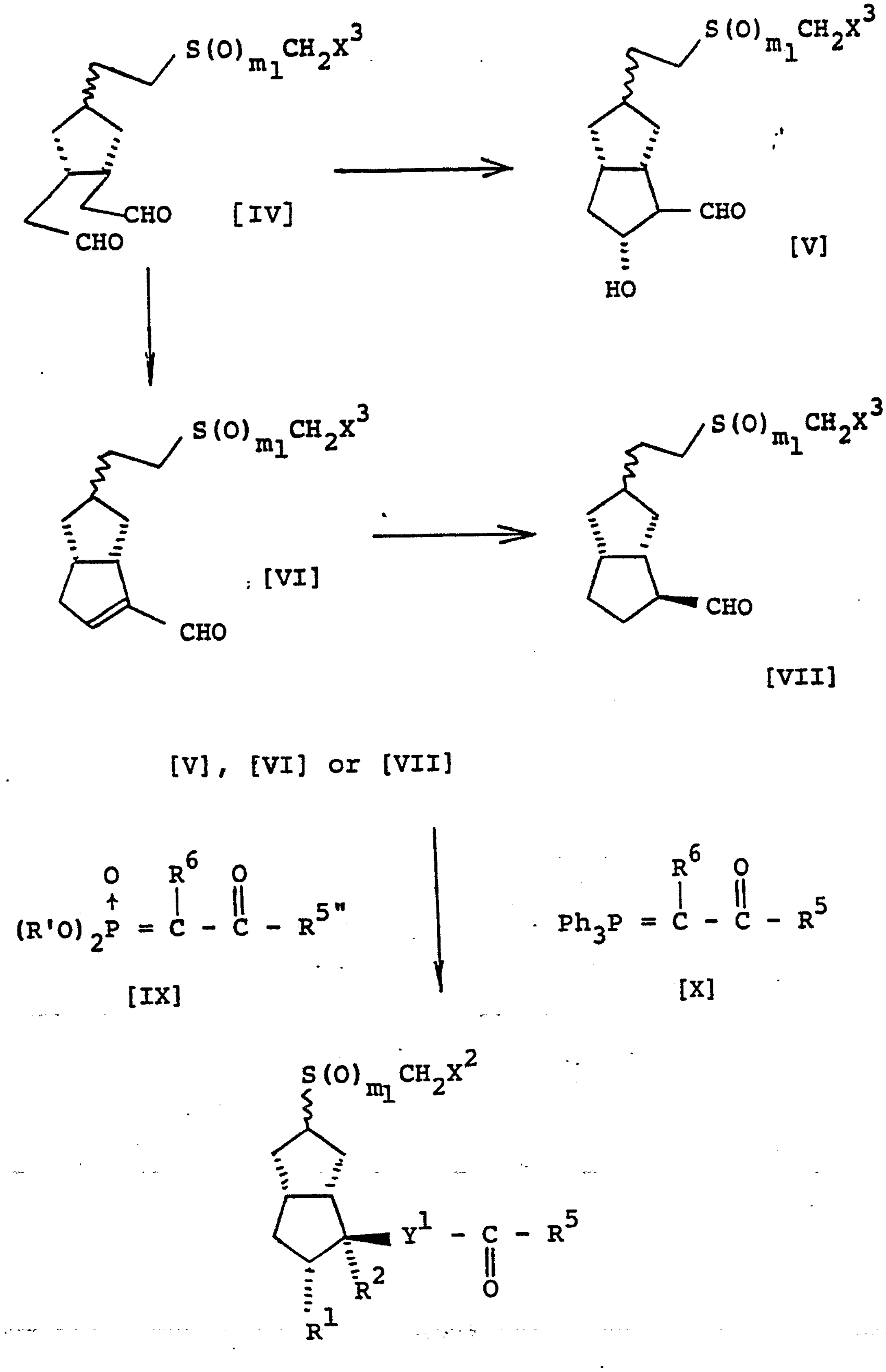

[IV]

[V]

[VI]

[VII]

[V], [VI] or [VII]

[IX]

[X]

[VIII]

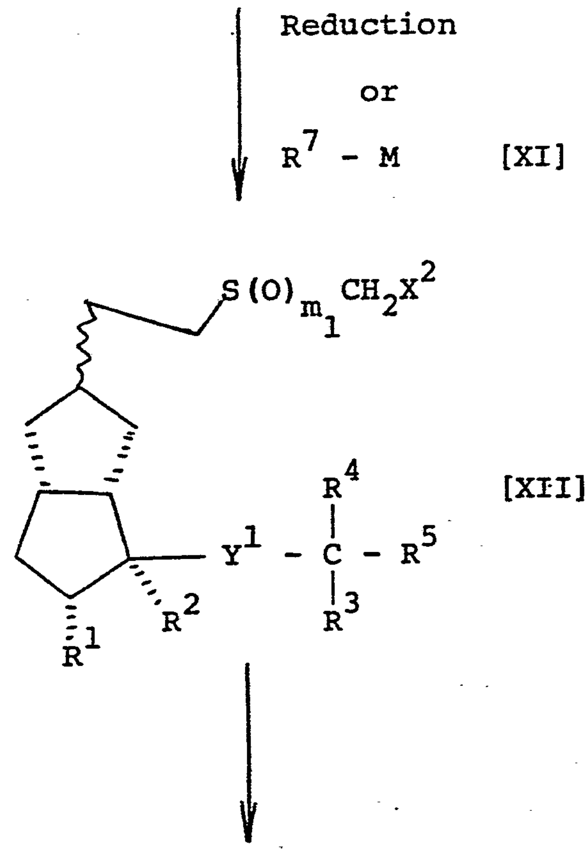


[XII]

[I]

Scheme-A

wherein $Y^1$, $R^1$, $R^2$, $R^3$, $R^4$, $R^5$, $R^6$ and m are each as defined above and $X^2$ is a free or esterified carboxyl group, a cyano group or a group of the formula:

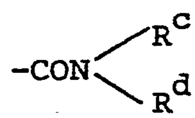

[wherein $R^c$ and $R^d$ are each independently a $C_1$-$C_4$ alkyl group, a $C_3$-$C_7$ cycloalkyl group, a benzyl group, a phenyl group, a phenyl group substituted with a $C_1$-$C_4$ alkyl group, or when taken together with the adjacent nitrogen atom to which they are attached, they represent

a 5 to 7 membered saturated heterocyclic group], $X^3$ is same as $X^2$ provided that $X^3$ is not a free carboxyl group. Ph is a phenyl group, $R^{5''}$ is same as $R^5$ provided that $R^{5''}$ is not a hydrogen atom. M is a lithium atom or a group of the formula: -Mg halo (halo is a halogen atom), $R^7$ is a $C_1$-$C_4$ alkyl group, $m_1$ is 1 or 2, and R' is a $C_1$-$C_4$ alkyl group.

Oxidation of the compound [III] into the dialdehyde [IV] can be accomplished by treating the former with sodium metaperiodate in the presence of a catalytic amount of osmium tetraoxide in an inert solvent at a temperature in the range from 0°C to room temperature. Example of the inert solvent include water, ethers (e.g. dioxane, THF) and aqueous ethers.

The dialdehyde [IV] can be also obtained by ozonization of the compound [III] at a temperature in the range from -80°C to -30°C, followed by reductive cleavage with dialkyl sulfide, triphenylphosphine, sodium bisulfite, zinc or the like, or by the catalytic hydrogenation in the presence of a catalyst (e.g. palladium on charcoal).

Example of the inert solvent for ozonization include alkanols (e.g. methanol, ethanol), halogenated hydrocarbon and esters (e.g. ethyl acetate). Reduction of an ozonide may be accomplished by a per se conventional procedure at the range from -30°C to 0°C.

Aldol condensation of the dialdehyde [IV] into an aldole derivative [V] is carried out in the presence

of an acid or a base in an inert solvent (e.g. water, alkanols, aqueous alkanols, ethers, esters) at a temperature in the range from -70°C to room temperature. Example of the suitable base are alkali hydroxide (e.g. potassium hydroxide, sodium hydroxide), alkali carbonate and alkali hydrogen carbonate.

The compound [VI] can be obtained by treating the dialdehyde [IV] with an acid or a base in an inert solvent at a temperature in the range from room temperature to the boiling temperature of the solvent.

The compound [VI] can be easily converted into the compound [VII] by conventional catalytic hydrogenation in the presence of a catalyst (e.g. palladium on charcoal), if necessary, followed by epimerization.

The compounds [V , VI , VII] can be easily converted into the compound [VIII] by reacting of the former with a compound [IX] or a compound [X] in an inert solvent (e.g. dioxane, ether, THF, dimethoxyethane, benzene, toluene, n-hexane, DMSO) at a temperature in the range from -10°C to 50°C, optionally followed by protection of a hydroxy group, hydrolysis of an ester group and/or reduction of a vinylene group.

The selective reduction of a compound of the formula [VIII']

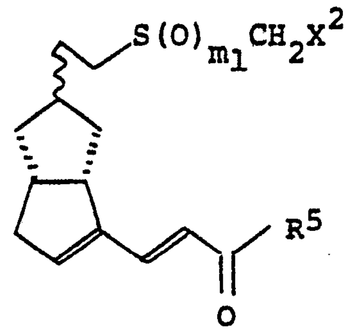

[VIII']

wherein $X^2$, $R^5$ and $m_1$ are each as defined above into a comppound of the formula [VIII"]

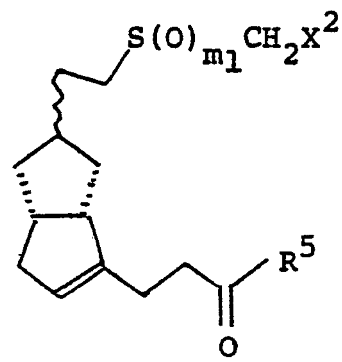

[VIII"]

wherein $X^2$, $R^5$ and $m_1$ are each as defined above, can be carried out by reacting the former with trialkylsilane in the presence of titanium tetrachloride in an inert solvent (e.g. halogenated hydrocarbon) at a temperature in the range from -78°C to 0°C.

The carbonyl compound [VIII] can be converted into the alcohol compound [XII] by reacting of the former with a reducing agent or an organometalic compound [XI].

The reduction can be carried out in an inert solvent (e.g. THF, ether, dimethoxyethane, pentane, hexane, benzene, toluene, methanol, ethanol) at a temperature in the range from -70°C to room temperature.

As the reducing agent, there may be used for example trialkylborohydride (e.g. lithium triisobutyl borohydride), bis (2,4,6-tri-tert-butylphenoxy) aluminum hydride, sodium borohydride, zinc borohydride, diisobutyl aluminum hydride, diisobutyl aluminum-2,6-di-tert-butyl-4-methyl-phenol, ethoxy 1,1'-binaphthyl-2,2'-dioxyaluminum lithium hydride etc.

The reaction of the carbonyl compound [VIII] with an organometalic compound [XI] can be carried out in an inert solvent (e.g. ether, THF, dioxane) at a temperature in the range from -70°C to room temperature. The organometalic compound [XI] can be prepared by conventional procedures.

If necessary, the compound [XII] can be converted into the compound [I] by oxidation of a sulfoxide group into a sulfone group, reduction of a sulfoxide group into a sulfide group, hydrolysis of an amide, ester or cyano group, esterification of a carboxyl group, amidation of a free or esterified carboxyl group, reduction of a vinylene group, protection of a hydroxy group and/or deprotection of a protected hydroxy group.

The protection and deprotection of a hydroxy group can be carried out by conventional procedure [Protective Group in Organic Chemistry, Edited by J.F.W. McOmie (1973) 95-143].

The reduction of a vinylene group can be accomplished by catalytic hydrogenation in an inert solvent (e.g. alkanol, aqueous alkanol) at a temperature in the range from 0°C to room temperature.

The oxidation of a sulfoxide group can be carried out by treating the sulfoxide compound with an oxidizing agent in an inert solvent (e.g. THF, dioxane, methanol, ethanol, dichloromethane chloroform, n-hexane, benzene, toluene) at a temperature in the range from 0°C to the boiling temperature of the solvent.

As the oxidizing agent, there may be used for example m-chloroperbenzoic acid, hydrogen peroxide, chromic acid, potassium permanganate, ozone etc.

The reduction of a sulfoxide group can be carried out by reacting of the sulfoxide compound with a reducing agent in an inert solvent (e.g. THF, methanol, ethanol, dichloromethane, n-hexane, benzene, toluene, water) at a temperature in the range from 0°C to the boiling temperature of the solvent.

As the reducing agent there may be used for example the hydride compound (e.g. lithium aluminum hydride, diborane, sodium borohydride, sodium cyanoborohydride) in the presence of an alkylating agent (e.g. tetramethylfluoroborate) or Lewis acid (e.g. titanium tetrachloride,

cobalt chloride), trichlorosilyl hydride, trimethyl silyliodide, hydrogen iodide, triphenyl-phosphine and carbon tetrachloride, iodine and sodium iodide, the thiol compound (e.g. thiophenol, benzylmercaptane, thioacetic acid), the thiol compound and trifluoroacetic acid, sodium bisulfite etc.

The steps of amidation of a carboxyl group, amidation of a esterified carboxy group, hydrolysis of an amide group into a carboxyl group, hydrolysis of an esterified carboxyl group and esterification of a carboxyl grop may be represented by the following scheme:

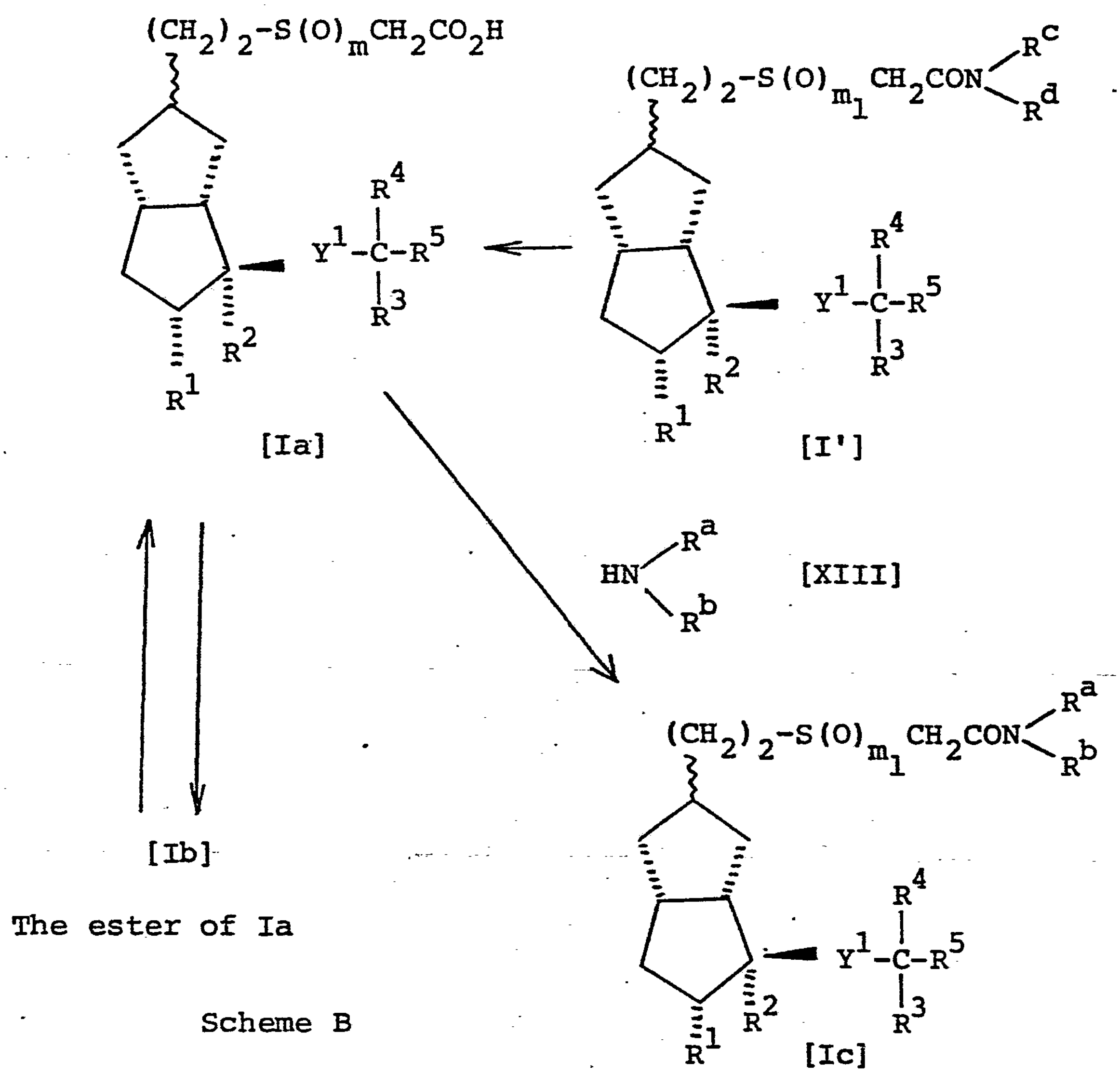

[Ia]

[I']

[XIII]

[Ib]

The ester of Ia

[Ic]

Scheme B

Amidation of a carboxyl group can be carried out by conventional procedure. For instance, it can be accomplished in an inert solvent (e.g. ether, THF) by treating a carboxyl compound [Ia] with an amine [XIII] ($R^a$ and $R^b$ are each as defined above) in the presence of dehydrolyzing agent (e.g. dicyclohexylcarbodiimide) at a temperature in the range from 0°C to room temperature, or by treating the functionally active derivative (e.g. mixed acid anhydride) of [Ia] with the amine [XIII] in an inert solvent (e.g. ether, THF, chloroform) at a temperature in the range from -10°C to room temperature.

Amidation of an esterified carboxyl group can be carried out by treating an ester compound [Ib] with the amine [XIII] in an inert solvent (e.g. DMF, methanol, ethanol, THF) at a temperature in the range from room temperature to the boiling temperature of the solvent.

Hydrolysis of a amido group into a carboxyl group can be carried out in the presence of an alkali (e.g. sodium hydroxide, potassium hydroxide) in an inert solvent (e.g. aqueous alkanol, DMSO) at a temperature in the range from 60°C to the boiling temperature of the solvent.

The hydrolysis of the ester compound [Ib] and the esterification of a carboxyl group can be carried out by conventional procedure.

The compound [III] used as an intermediate in the method-A can be prepared from a thiol compound [XIV] or an alcohol compound [XV],

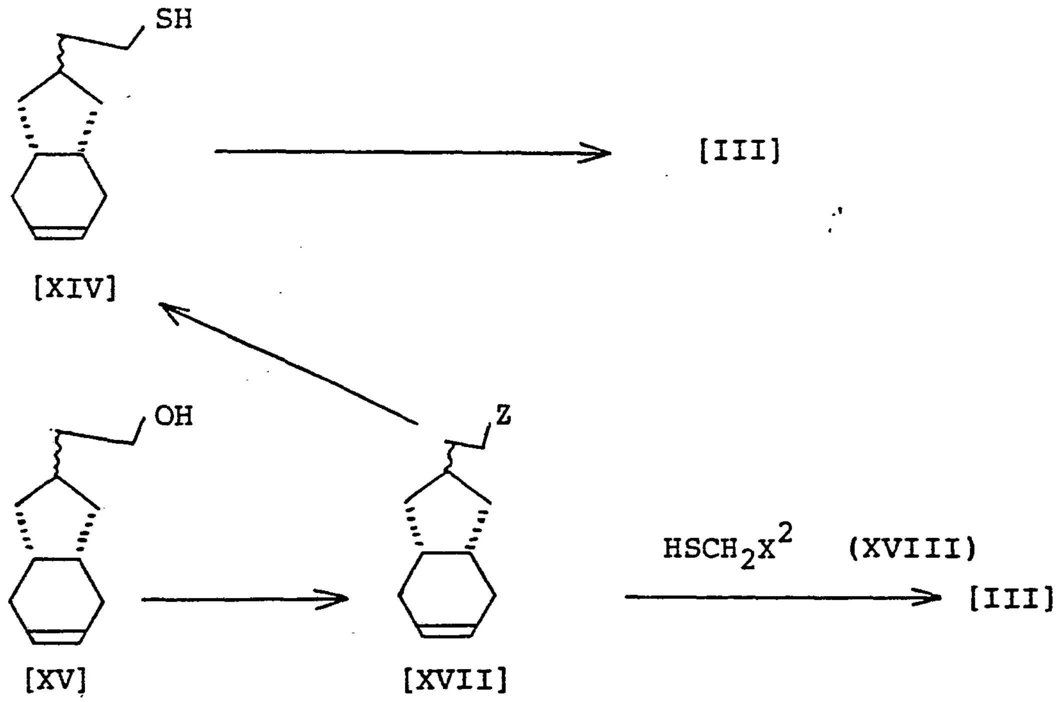


wherein $X^2$ is as defined above and Z is a halogen atom, a $C_1$-$C_4$ alkylsulfonyloxy group or an arylsulfonyloxy group.

The thiol compound [XIV] can be converted into the compound [III] by altylating the former with halogeno acetonitrile, halogeno acetate, halogeno acetic acid or halogeno compound of the formula:

$$\text{halo-}CH_2CON\begin{matrix} R^c \\ R^d \end{matrix} \qquad [XVI]$$

wherein halo is a halogen atom, and $R^c$ and $R^d$ are each as defined above, optionally followed by esterification of a carboxyl group.

The alkylation can be carried out in the presence of an alkali (e.g. alkali metal hydride, alkali metal amide, alkali metal, alkali metal hydroxide, t-butoxy

alkali metal) in an inert solvent (e.g. benzene, toluene, xylene, DMF, DMSO, alkanol) at a temperature in the range from 0°C to the boiling temperature of the solvent.

The esterification can be carried out by conventional procedure.

The compound [III] can be also prepared from the alcohol [XV] via a compound [XVII].

The sulfonylation of the alcohol compound [XV] can be carried out by treating of the alcohol [XV] with an alkylsulfonyl halide (e.g. methylsulfonyl chloride), an arylsulfonyl halide (e.g. p-toluenesulfonyl chloride) in the presence of a base (e.g. triethylamine, pyridine) in an inert solvent (e.g. n-hexane, benzene, toluene, ether, THF, dichloromethane) at a temperature in the range from 0°C to room temperature.

The halogenation of the alcohol compound [XV] can be easily carried out by using conventional halogenating agent (e.g. hydrogen bromide, thionyl chloride, phosphonium tribromide, phosphonium trichloride, triphenylphosphine-carbon tetrachloride).

The reaction of the compound [XVII] with the thiol compound (XVIII) can be carried out in the presence of an alkali (e.g. alkali metal hydride, alkali metal amide, alkali metal, alkali metal hydroxide, t-butoxy alkali metal) in an inert solvent (e.g. benzene, toluene, xylene, DMF, DMSO, alkanol) at a temperature in the range from 0°C to the boiling temperature of the solvent.

The compound [XIV] can be prepared from the

compound [XVII] by reacting of the latter with potassium thioacetate, sodium thiobenzoate, sodium hydrosulfite or thiourea in an inert solvent (e.g. alkanol, aqueous alkanol, DMF, DMSO, toluene, benzene, THF) at a temperature in the range from room temperature to the boiling temperature of the solvent, optionally followed by hydrolysis of an ester group.

The alcohol compound [XV] can be prepared by way of the following three paths shown in the Scheme C, D, and E.

The compound [XV] can be obtained from the carbonyl compound [XIX] by the process shown in the Scheme C.

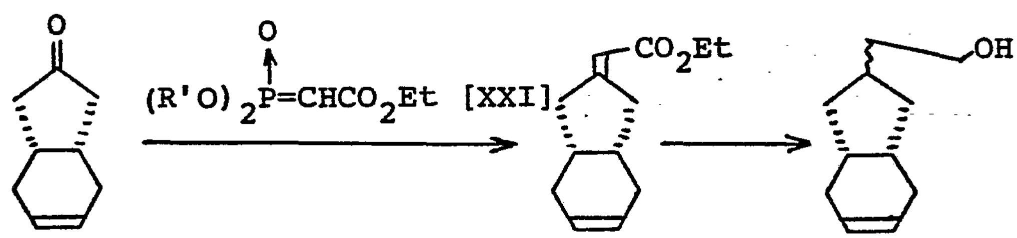


[XIX] [XX] [XV]

Scheme C

The compound [XIX] can be easily converted into the ester compound [XX] by reacting of the former with a compound [XXI] in an inert solvent (e.g. dioxane, ether, THF, dimethoxyethane, benzene, DMSO) at a temperature in the range from -10°C to 50°C.

The compound [XX] can be easily converted into the alcohol compound [XVI] by Birth reduction of the

former according to the known procedure [J. Org. Chem., 37 2871 (1972)]. The reduction can be accomplished by treating the ester [XX] with lithium or sodium in the presence of liquid ammonia and alcohol (e.g. methanol, ethanol) in an inert solvent (e.g. ether, dioxane) at a temperature in the range from -70° to -40°C.

The compound [XV] can be obtained from the compound [XIX] also by the process shown in the Scheme D.

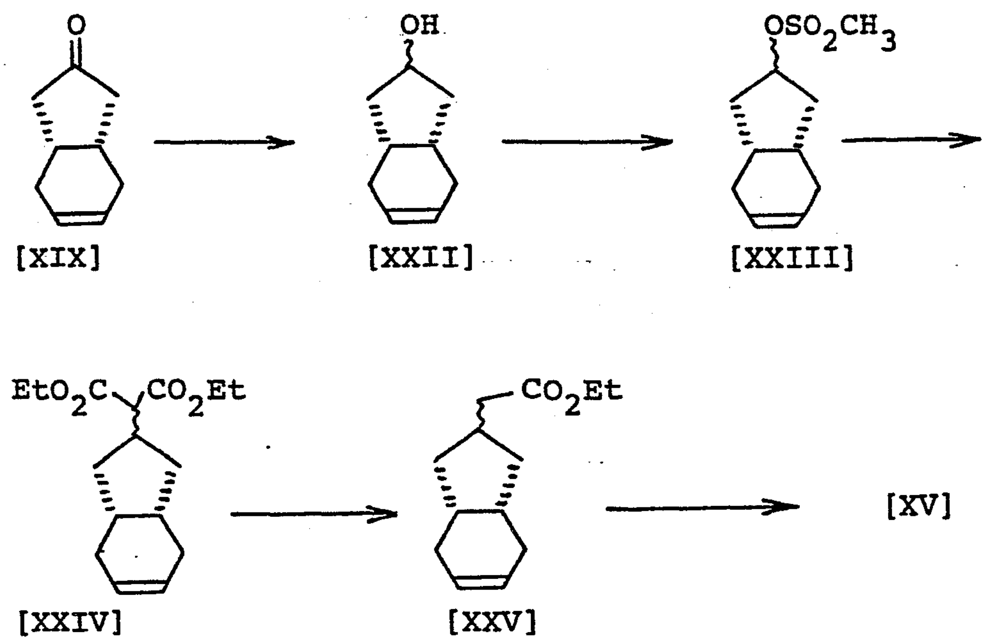

[XIX] [XXII] [XXIII]

[XXIV] [XXV] [XV]

Scheme D

Reduction of the carbonyl compound [XIX] into the alcohol compound [XXII] can be easily carried out by a conventional procedure. The compound [XXII] can be converted into the compound [XXIII] by reacting the former with methanesulfonyl chloride and triethylamine in an inert solvent (e.g. THF) at a temperature in the range from 0°C to 40°C.

The compound [XXIII] can be converted into the diester compound [XXIV] by reacting the former with diethylmalonate in the presence of sodium ethoxide in ethanol at a temperature in the range from 50°C to boiling point of the solvent. Decarboxylation of the diester [XXIV] into the ester compound [XXV] can be carried out by heating of the former in the presence of sodium chloride in an inert solvent (e.g. DMSO) at a temperature in the range from 80°C to a boiling point of the solvent.

Reduction of the ester [XXV] into the alcohol [XV] can be accomplished by reacting with lithium aluminum hydride or lithium trimethoxy aluminum hydride in an inert solvent (e.g. THF) at a temperature in the range from 0°C to a boiling point of the solvent.

The compound [XV] can be obtained also by the process shown in the Scheme E.

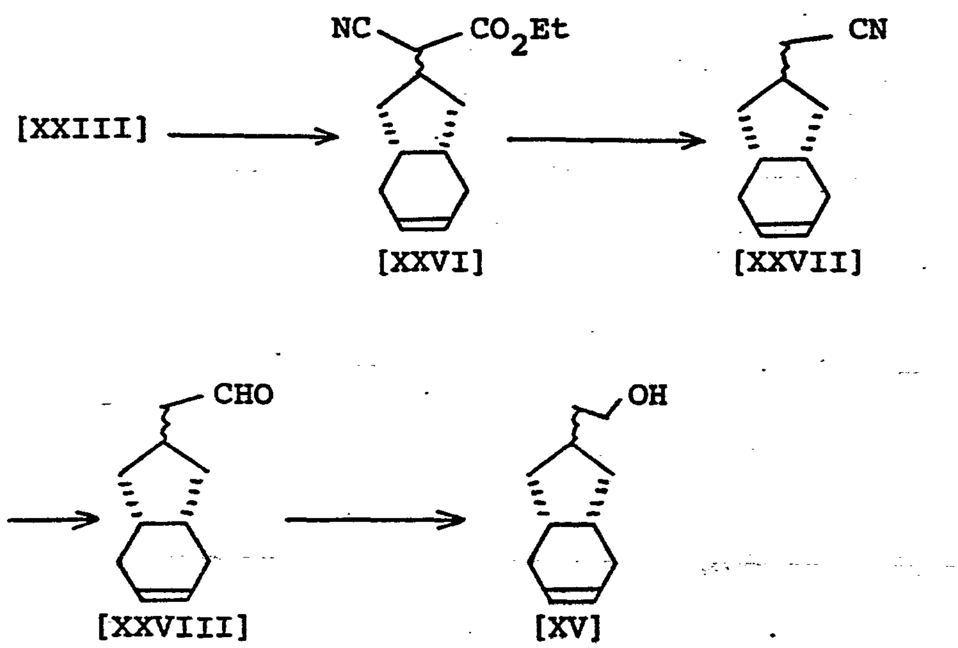

[XXIII]

[XXVI]

[XXVII]

[XXVIII]

[XV]

Scheme E

The compound [XXIII] can be converted into the compound [XXVI] by reacting of the former with ethyl cyanoacetate in the presence of sodium ethoxide in ethanol at a temperature in the range from 50°C to a boiling point of the solvent. Decarboxylation of the ester [XXVI] into the compound [XXVII] can be carried out by heating in the presence of sodium chloride and water in an inert solvent (e.g. DMSO) at a temperature in the range from 80°C to a boiling point of the solvent.

Reduction of the nitrile [XXVII] into the aldehyde [XXVIII] can be accomplished by reacting with diisobutyl aluminum hydride in an inert solvent (e.g. THF, ether, hexane) at a temperature in the range from -70°C to ambient temperature. Reduction of the aldehyde [XXVIII] into the alcohol [XV] can be easily carried out by a conventional procedure.

(B) The bicyclooctane compound [I] can be also prepared from a carbonyl compound [XXIX] by the process shown in the following scheme-

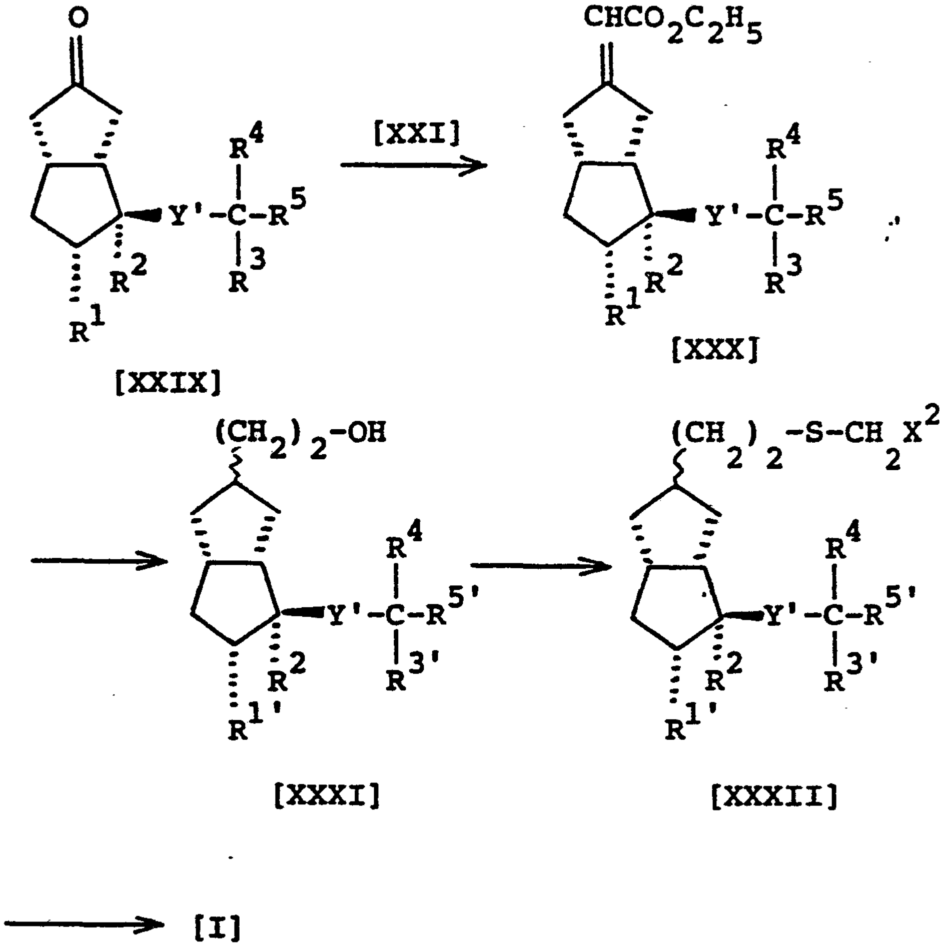


[XXIX] [XXX] [XXXI] [XXXII] [I]

wherein Y', $R^1$, $R^2$, $R^3$, $R^4$ and $R^5$ are each as defined above, $R^{1'}$ is a hydrogen atom or a protected hydroxyl group, or $R^{1'}$ and $R^2$, when taken together, mean a single linkage to form a double bond between the carbon atoms to which they are linked, $R^{3'}$ is a protected hydroxy group and $R^{5'}$ is a hydrogen atom, a $C_1$-$C_{12}$ alkyl group, a $C_2$-$C_{12}$ alkenyl group, a $C_2$-$C_{12}$ alkynyl group, a $C_3$-$C_{10}$ cycloalkyl group, a $C_4$-$C_{10}$ cycloalkenyl group, a protected hydroxy $C_1$-$C_{12}$ alkyl group, a $C_3$-$C_{10}$ heterocyclic group, a phenyl group optionally substituted with a halogen atom, a protected hydroxyl group, a $C_1$-$C_4$ alkyl group, a trifluoromethyl group or a $C_1$-$C_4$ alkoxy group or a formula:

A-B' (A is a $C_1$-$C_7$ alkylene chain and B' is a $C_3$-$C_{10}$ cycloalkyl group, a $C_4$-$C_{10}$ cycloalkenyl group, a $C_1$-$C_{12}$ alkoxy group, a $C_3$-$C_{10}$ cycloalkoxy group, a $C_4$-$C_{10}$ cycloalkenyloxy group, a $C_3$-$C_{10}$ heterocyclic group, or a phenyl or phenoxy group, which phenyl or phenoxy group is optionally substituted with a halogen atom, a protected hydroxy group, a $C_1$-$C_4$ alkyl group, a trifluoromethyl group or a $C_1$-$C_4$ alkoxy group.

The compound [XXIX] can be converted into the alcohol compound [XXXI] via the ester compound according to the known procedure [Japanese Patent Application Kokai (Laid-open) No. Sho 59-141536 (1984)].

The reactions from the alcohol compound [XXXI] into the compound [XXXII] can be carried out by the same procedure as used in the synthesis of the compound [III] from the compound [XV].

If necessary, the compound [XXXII] can be easily converted into the compound [I] by oxidation of a sulfide group, hydrolysis of an amide, ester or cyano group, esterification of a carboxyl group, amidation of a free or esterified carboxyl group, reduction of a vinylene group, protection of a hydroxy group and/or deprotection of a protected hydroxy group.

The compound [XXIX] used as an intermediate in the method-B can be prepared according to the known procedure [Japanese Patent Application Kokai (Laid-open) No. Sho 59-141536 (1984)].

C) The bicyclooctane compound [I] can be also prepared from the thiol compound of the formula:

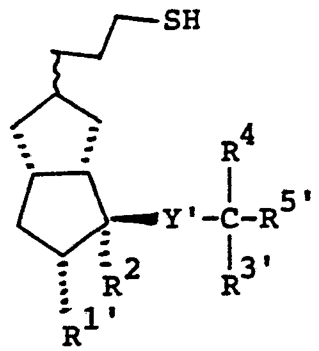

[XXXIII]

wherein $R^{1'}$, $R^2$, $R^{3'}$, $R^4$, $R^{5'}$ and Y' are each as defined above, by reacting of the latter with the halogeno compound of the formula:

$$halo\text{-}CH_2\text{-}X^2 \qquad [XXXIV]$$

wherein halo is a halogen atom and $X^2$ is as defined above, optionally followed by oxidation of a sulfide group, hydrolysis of an amide, ester or cyano group, esterification of a carboxyl group, amidation of a free or esterified carboxyl group, reduction of a vinylene group, protection of a hydroxy group and/or deprotection of a protected hydroxy group.

The reaction between the thiol compound [XXXIII] and the halogeno compound [XXXIV] can be carried out in the presence of an alkali (e.g. alkali metal hydride, alkali metal amide, alkali metal, t-butoxy alkali metal) at a temperature in the range from 0°C to the boiling temperature of the solvent. Example of the suitable solvent is benzene, toluene, xylene, DMF, DMSO or alkanol.

The compound [XXXIII] used as an intermediate

in the method-C can be prepared from the alcohol compound [XXXI] by the same procedure as used in the synthesis of the compound [XIV] from the compound [XV].

According to the present invention, the four stereoisomers of the formulae:

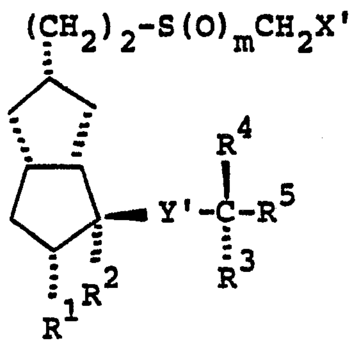

[A]

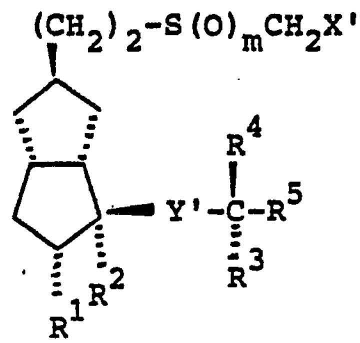

[B]

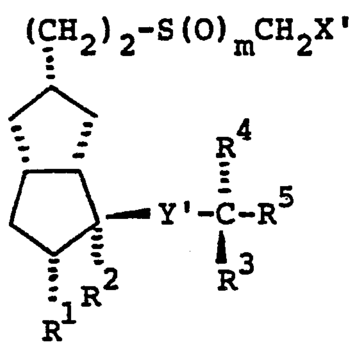

[C]

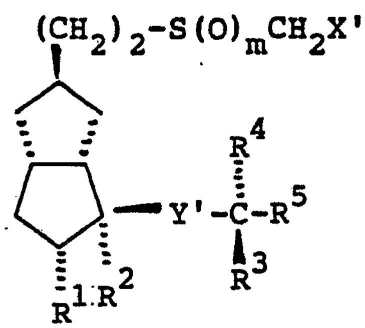

[D]

can be prepared.

In general, the bicyclooctane compound [I] can be obtained as a mixture of these stereoisomers which can be easily separated by the conventional method (e.g. column chromatography) with high purity.

If necessary, it is possible to yield selectively the bicyclooctane compound [I] of either one of these stereoisomers by changing the kinds and properties of solvents, reaction temperature, the organometalic compounds [XI] and reducing agents.

Furthermore, bicyclooctane compound [I] can be separated into optical isomers by a conventional method.

Among the bicyclooctane compounds [I] thus obtained, the compound [Ia] can be converted to its pharmacologically acceptable salt form. The pharmaceutically acceptable salts of these bicyclooctane compounds are those with pharmaceutically acceptable metal cation such as, sodium, potassium, magnesium and calcium, ammonium or amine cations.

If necessary, in order to improve the solubility in water, the bicyclooctane compounds [I] can be converted into inclusion compounds with some kinds of cyclodextrins.

The preparation of pharmaceutical compositions can be carried out by a conventional method, for example, the bicyclooctane compounds [I], they may be mixed with carriers, diluents, lubricants, fillers and/or binders such as lactose, sucrose, calcium, phosphate, starch, talcum, casein, magnesium stearate, methyl cellulose, polyglycols, tragacanth and the like, sometimes together with stabilizers and emulsifying agents. The resulting mixture may be processed in a usual manner to tablets, capsules, pills, ampoules and the like.

In a clinical practice, the bicyclooctane compounds [I] can be administered orally, subcutaneously, intravenously, intramuscularly or the like. In general, the oral administration is preffered.

The daily dosage may vary depending upon the administration route and the usual oral dosage of the active ingredient is between about 0.1 mg and about 100 mg daily for human beings.

Specific examples of the bicyclooctane compound [I] are as follows. Every compounds below has four isomers, that is, (3'α, 7α), (3'α, 7β), (3'β, 7α) and (3'β, 7β).

2β-(3'-hydroxy-trans-1'-octenyl)-3α-hydroxy-7-(2'-carboxymethylthioethyl)-cis-bicyclo[3,3,0]octane

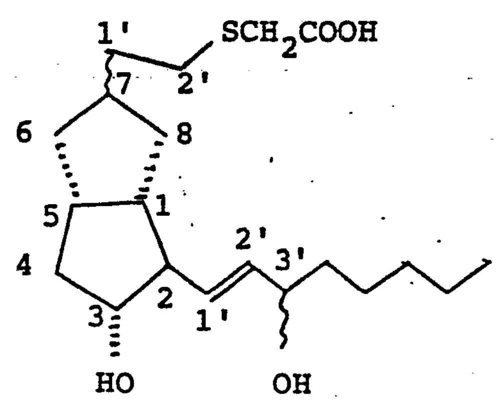

2β-(3'-hydroxy-trans-1'-nonenyl)-3α-hydroxy-7-(2'-carboxymethylthioethyl)-cis-bicyclo[3,3,0]octane

2β-(3'-hydroxy-5'-methyl-trans-1'-nonenyl)-3α-hydroxy-7-(2'-carboxymethylthioethyl)-cis-bicyclo[3,3,0]-octane

2β-(3'-hydroxy-4',4'-dimethyl-trans-1'-octenyl)-3α-hydroxy-7-(2'-carboxymethylthioethyl)-cis-bicyclo-[3,3,0]octane

2β-(3'-hydroxy-4'-methyl-trans-1'-pentenyl)-3α-hydroxy-7-(2'-carboxymethylthioethyl)-cis-bicyclo-[3,3,0]octane

2β-(3'-hydroxy-4'-methyl-trans-1'-octenyl)-3α-hydroxy-7-(2'-carboxymethylthioethyl)-cis-bicyclo[3,3,0]-octane

2β-(3'-hydroxy-5',9'-dimethyl-trans-1',8'-decadienyl)-3α-hydroxy-7-(2'-carboxymethylthioethyl)-cis-bicyclo[3,3,0]octane

2β-(3'-hydroxy-4'-methylene-trans-1'-octenyl)-3α-hydroxy-7-(2'-carboxymethylthioethyl)-cis-bicyclo-[3,3,0]octane

2β-(3'-hydroxy-4',4',9'-trimethyl-trans-1',8'-decadienyl)-3α-hydroxy-7-(2'-carboxymethylthioethyl)-cis-bicyclo[3,3,0]octane

2β-(3'-hydroxy-trans-1'-cis-5'-octadienyl)-3α-hydroxy-7-(2'-carboxymethylthioethyl)-cis-bicyclo[3,3,0]-octane

2β-(3'-hydroxy-4'-methyl-trans-1'-octen-6'-ynyl)-3α-hydroxy-7-(2'-carboxymethylthioethyl)-cis-bicyclo[3,3,0]octane

2β-(3'-hydroxy-trans-1'-decen-9'-ynyl)-3α-hydroxy-7-(2'-carboxymethylthioethyl)-cis-bicyclo[3,3,0]-octane

2β-(3'-hydroxy-5'-methyl-trans-1'-decen-8'-

ynyl)-3α-hydroxy-7-(2'-carboxymethylthioethyl)-cis-bicyclo[3,3,0]octane

2β-(3'-hydroxy-trans-1'-octen-5'-ynyl)-3α-hydroxy-7-(2'-carboxymethylthioethyl)-cis-bicyclo[3,3,0]-octane

2β-(3'-hydroxy-3'-cyclopentyl-trans-1'-propenyl)-3α-hydroxy-7-(2'-carboxymethylthioethyl)-cis-bicyclo-[3,3,0]octane

2β-(3'-hydroxy-3'-cyclohexyl-trans-1'-propenyl-3α-hydroxy-7-(2'-carboxymethylthioethyl)-cis-bicyclo-[3,3,0]octane

2β-(3'-hydroxy-3'-(1"-adamantyl)-trans-1'-propenyl)-3α-hydroxy-7-(2'-carboxymethylthioethyl)-cis-bicyclo[3,3,0]octane

2α-(3'-hydroxy-3'-(norbornan-2"-yl)-trans-1'-propenyl)-3α-hydroxy-7-(2'-carboxymethylthioethyl)-cis-bicyclo[3,3,0]octane

2β-(3'-hydroxy-3'-(hexahydroindan-2'-yl)-trans-1'-propenyl)-3α-hydroxy-7-(2'-carboxymethylthioethyl)-cis-bicyclo[3,3,0]octane

2β-(3'-hydroxy-3'-(3"-ethylcyclopentyl)-trans-1'-propenyl)-3α-hydroxy-7-(2'-carboxymethylthioethyl)-cis-bicyclo[3,3,0]octane

2β-(3'-hydroxy-3'-(4"-methylcyclohexyl)-trans-1'-propenyl)-3α-hydroxy-7-(2'-carboxymethylthioethyl)-cis-bicyclo[3,3,0]octane

2β-(3'-hydroxy-3'-(cyclohexen-4"-yl)-trans-1'-propenyl)-3α-hydroxy-7-(2'-carboxymethylthioethyl)-cis-bicyclo[3,3,0]octane

2β-(3'-hydroxy-3'-(cyclopenten-4"-yl)-trans-1'-propenyl)-3α-hydroxy-7-(2'-carboxymethylthioethyl)-cis-bicyclo[3,3,0]octane

2β-(3'-hydroxy-3'-(bicyclo[4,3,0]non-3"-en-8"-yl)-trans-1'-propenyl)-3α-hydroxy-7-(2'-carboxymethylthioethyl)-cis-bicyclo[3,3,0]octane

2β-(3'-hydroxy-3'-(norbornen-5"-yl)-trans-1'-propenyl)-3α-hydroxy-7-(2'-carboxymethylthioethyl)-cis-bicyclo[3,3,0]octane

2β-(3',8'-dihydroxy-trans-1'-octenyl)-3α-hydroxy-7-(2'-carboxymethylthioethyl)-cis-bicyclo[3,3,0]-octane

2β-(3',8'-dihydroxy-trans-1'-nonenyl)-3α-hydroxy-7-(2'-carboxymethylthioethyl)-cis-bicyclo[3,3,0]-octane

2β-(3',9'-dihydroxy-trans-1'-nonenyl)-3α-hydroxy-7-(2'-carboxymethylthioethyl)-cis-bicyclo[3,3,0]-octane

2β-(3',8'-dihydroxy-5'-methyl-tran-1'-octenyl-3α-hydroxy-7-(2'-carboxymethylthioethyl)-cis-bicyclo-[3,3,0]octene

2β-(3'-hydroxy-3'-(2",3"-dihydrobenzofuran-2"-yl)-trans-1'-propenyl)-3α-hydroxy-7-(2'-carboxymethylthioethyl)-cis-bicyclo[3,3,0]octane

2β-(3'-hydroxy-3'-(1",4"-dihydrobenzodioxan-2"-yl)-trans-1'-propenyl)-3α-hydroxy-7-(2'-carboxymethylthioethyl)-cis-bicyclo[3,3,0]octane

2β-(3'-hydroxy-3'-(thiophen-2"-yl)-trans-1'-propenyl)-3α-hydroxy-7-(2'-carboxymethylthioethyl)-cis-bicyclo[3,3,0]octane

2β-(3'-hydroxy-3'-(pyridin-3"-yl)-trans-1'-propenyl)-3α-hydroxy-7-(2'-carboxymethylthioethyl)-cis-bicyclo[3,3,0]octane

2β-(3'-hydroxy-3'-phenyl-trans-1'-propenyl)-3α-hydroxy-7-(2'-carboxymethylthioethyl)-cis-bicyclo[3,3,0]-octane

2β-(3'-hydroxy-3'-(3"-chlorophenyl)-trans-1'-propenyl)-3α-hydroxy-7-(2'-carboxymethylthioethyl)-cis-bicyclo[3,3,0]octane

2β-(3'-hydroxy-3'-(4"-fluorophenyl)-trans-1'-propenyl)-3α-hydroxy-7-(2'-carboxymethylthioethyl)-cis-bicyclo[3,3,0]octane

2β-(3'-hydroxy-3'-(3"-hydroxyphenyl)-trans-1'-propenyl)-3α-hydroxy-7-(2'-carboxymethylthioethyl)-cis-bicyclo[3,3,0]octane

2β-(3'-hydroxy-3'-(toluene-3"-yl)-trans-1'-propenyl)-3α-hydroxy-7-(2'-carboxymethylthioethyl)-cis-bicyclo[3,3,0]octane

2β-(3'-hydroxy-3'-(3"-trifluoromethylphenyl)-trans-1'-propenyl)-3α-hydroxy-7-(2'-carboxymethylthioethyl)-cis-bicyclo[3,3,0]octane

2β-(3'-hydroxy-3'-(3"-methoxyphenyl)-trans-1'-propenyl)-3α-hydroxy-7-(2'-carboxymethylthioethyl)-cis-bicyclo[3,3,0]octane

2β-(3'-hydroxy-3'-(3",4"-dimethoxyphenyl)-trans-1'-propenyl)-3α-hydroxy-7-(2'-carboxymethylthioethyl)-cis-bicyclo[3,3,0]octane

2β-(3'-hydroxy-4'-(1"-adamantly)-trans-1'-butenyl)-3α-hydroxy-7-(2'-carboxymethylthioethyl)-cis-bicyclo[3,3,0]octane

2β-(3'-hydroxy-4'-cyclopentyl-trans-1'-butenyl-3α-hydroxy-7-(2'-carboxymethylthioethyl)-cis-bicyclo[3,3,0]-octane

2β-(3'-hydroxy-4'-cyclohexyl-trans-1'-butenyl)-3α-hydroxy-7-(2'-carboxymethylthioethyl)-cis-bicyclo-[3,3,0]octane

2β-(3'-hydroxy-4'-(3"-ethylcyclopentyl)-trans-1'-butenyl)-3α-hydroxy-7-(2'-carboxymethylthioethyl)-cis-bicyclo[3,3,0]octane

2β-(3'-hydroxy-4'-(hexahydroindan-2"-yl)-trans-1'-butenyl)-3α-hydroxy-7-(2'-carboxymethylthioethyl)-cis-bicyclo[3,3,0]octane

2β-(3'-hydroxy-4'-(cyclohexen-4"-yl)-trans-1'-butenyl)-3α-hydroxy-7-(2'-carboxymethylthioethyl)-cio-bicyclo[3,3,0]octane

2β-(3'-hydroxy-4'-(cyclopenten-4"-yl)-trans-1-butenyl)-3α-hydroxy-7-(2'-carboxymethylthioethyl)-cis-bicyclo[3,3,0]octane

2β-(3'-hydroxy-4'-(bicyclo[4,3,0]non-3"-en-8"-yl)-trans-1'-butenyl)-3α-hydroxy-7-(2'-carboxymethylthioethyl)-cis-bicyclo[3,3,0]octane

2β-(3'-hydroxy-4'-(norbornen-5"-yl)-trans-1'-butenyl)-3α-hydroxy-7-(2'-carboxymethylthioethyl)-cis-bicyclo[3,3,0]octane

2β-(3'-hydroxy-4'-(imidazol-1"-yl)-trans-1'-butenyl)-3α-hydroxy-7-(2'-carboxymethylthioethyl-cis-bicyclo[3,3,0]octane

2β-(3'-hydroxy-4'-(pyridin-3"-yl)-trans-1'-butenyl)-3α-hydroxy-7-(2'-carboxymethylthioethyl)-cis-bicyclo[3,3,0]octane

2β-(3'-hydroxy-4'-(indol-3"-yl)-trans-1'-butenyl)-3α-hydroxy-7-(2'-carboxymethylthioethyl)-cis-bicyclo[3,3,0]octane

2β-(3'-hydroxy-4'-(2"-pyrrolidon-1'-yl)-trans-1'-butenyl)-3α-hydroxy-7-(2'-carboxymethylthioethyl)-cis-bicyclo[3,3,0]octane

2β-(3'-hydroxy-4'-(thiophen-3"-yl)-trans-1'-butenyl)-3α-hydroxy-7-(2'-cargoxymethylthioethyl)-cis-bicyclo[3,3,0]octane

2β-(3'-hydroxy-4'-(1",2",2",6",6"-pentamethylpiperidin-4"-yl)-trans-butenyl)-3α-hydroxy-7-(2'-carboxymethylthioethyl)-cis-bicyclo[3,3,0]octane

2β-(3'-hydroxy-5'-ethoxy-trans-1'-pentenyl)-3α-hydroxy-7-(2'-carboxymethylthioethyl)-cis-bityclo[3,3,0]-octane

2β-(3'-hydroxy-8'-methoxy-trans-1'-octenyl)-3α-hydroxy-7-(2'-carboxymethylthioethyl)-cis-bicyclo[3,3,0]-octane

2β-(3'-hydroxy-7'-methoxy-trans-1'-heptenyl)-3α-hydroxy-7-(2'-carboxymethylthioethyl)-cis-bicyclo-[3,3,0]octane

2β-(3'-hydroxy-4'-propoxy-trans-1'-butenyl)-3α-hydroxy-7-(2'-carboxymethylthioethyl)-cis-bicyclo[3,3,0]-octane

2β-(3'-hydroxy-5'-methyl-7'-isopropoxy-trans-1'-heptenyl)-3α-hydroxy-7-(2'-carboxymethylthioethyl)-cis-bicyclo[3,3,0]octane

2β-(3'-hydroxy-4',4'-dimethyl-5'-ethoxy-trans-1'-pentenyl)-3α-hydroxy-7-(2'-carboxymethylthioethyl)-cis-bicycl [3,3,0]octane

2β-(3'-hydroxy-5'-cyclopentyloxy-trans-1'-pentenyl)-3α-hydroxy-7-(2'-carboxymethylthioethyl)-cis-bicyclo[3,3,0]octane

2β-(3'-hydroxy-4'-cyclohexyloxy-trans-1'-butenyl)-3α-hydroxy-7-(2'-carboxymethylthioethyl)-cis-bicyclo[3,3,0]octane

2β-(3'-hydroxy-4'-(1-menthoxy)-trans-1'-butenyl)-3α-hydroxy-7-(2'-carboxymethylthioethyl)-cis-bicyclo-[3,3,0]octane

2β-(3'-hydroxy-4'-(hexahydroindan-2"-yloxy)-trans-1'-butenyl)-3α-hydroxy-7-(2'-carboxymethylthioethyl)-cis-bicyclo[3,3,0]octane

2β-(3'-hydroxy-4'-(cyclohexen-4"-yloxy)-trans-1'-butenyl)-3α-hydroxy-7-(2'-carboxymethylthioethyl)-cis-bicyclo[3,3,0]octane

2β-(3'-hydroxy-4'-(cyclopenten-4"-yloxy)-trans-1'-butenyl)-3α-hydroxy-7-(2'-carboxymethylthioethyl)-cis-bicyclo[3,3,0]octane

2β-(3'-hydroxy-4'-(bicyclo[4,3,0]non-3"-en-8"-

yloxy)-trans-1'-butenyl)-3α-hydroxy-7-(2'-carboxymethylthioethyl)-cis-bicyclo[3,3,0]octane

2β-(3'-hydroxy-4'-phenyl-trans-1'-butenyl)-3α-hydroxy-7-(2'-carboxymethylthioethyl)-cis-bicyclo[3,3,0]-octane

2β-(3'-hydroxy-7'-phenyl-trans-1'-heptenyl)-3α-hydroxy-7-(2'-carboxymethylthioethyl)-cis-biciclo[3,3,0]-octane

2β-(3'-hydroxy-4'-(3"-methoxyphenyl)-trans-1'-butenyl)-3α-hydroxy-7-(2'-carboxymethylthioethyl)-cis-bicyclo[3,3,0]octane

2β-(3'-hydroxy-5'-(toluen-3"-yl)-trans-1'-pentenyl)-3α-hydroxy-7-(2'-carboxymethylthioethyl)-cis-bicyclo[3,3,0]octane

2β-(3'-hydroxy-4'-(2"-ethylphenyl)-trans-1'-butenyl)-3α-hydroxy-7-(2'-carboxymethylthioethyl)-cis-bicyclo[3,3,0]octane

2β-(3'-hydroxy-7'-(4"-hydroxyphenyl)-trans-1'-heptenyl)-3α-hydroxy-7-(2'-carboxymethylthioethyl)-cis-bicyclo[3,3,0]octane

2β-(3'-hydroxy-4'-phenoxy-trans-1'-butenyl)-3α-hydroxy-7-(2'-carboxymethylthioethyl)-cis-bicyclo[3,3,0]-octane

2β-(3'-hydroxy-6'-phenoxy-trans-1'-hexenyl)-3α-hydroxy-7-(2'-carboxymethylthioethyl)-cis-bicyclo[3,3,0]-octane

2β-(3'-hydroxy-5'-phenoxy-trans-1'-pentenyl)-3α-hydroxy-7-(2'-carboxymethylthioethyl)-cis-biciclo-

[3,3,0]octane

2β-(3'-hydroxy-4'-(4"-fuluorophenoxy)-trans-1'-butenyl)-3α-hydroxy-7-(2'-carboxymethylthioethyl)-cis-bicyclo[3,3,0]octane

2β-(3'-hydroxy-4'-(3"-trifuluoromethylphenoxy-trans-1'-butenyl)-3α-hydroxy-7-(2'-carboxymethylthioethyl)-cis-bicyclo[3,3,0]octane

2β-(3'-hydroxy-4'-(3"-chlorophenoxy)-trans-1'-butenyl)-3α-hydroxy-7-(2'-carboxymethylthioethyl)-cis-bicyclo[3,3,0]octane

2β-(3'-hydroxy-4'-(3"-methoxyphenoxy)-trans-1'-butenyl)-3α-hydroxy-7-(2'-carboxymethylthioethyl)-cis-bicyclo[3,3,0]octane

2β-(3'-hydroxy-4'-(toluen-3"-yloxy)-trans-1'-butenyl)-3α-hydroxy-7-(2'-carboxymethylthioethyl)-cis-bicyclo[3,3,0]octane

2β-(3'-hydroxy-4'-(3"-hydroxyphenoxy)-trans-1'-butenyl)-3α-hydroxy-7-(2'-carboxymethylthioethyl)-cis-bicyclo[3,3,0]octane

2β-(3'-hydroxy-4'-(3",4"-dimethoxyphenoxy)-trans-1'-butenyl)-3α-hydroxy-7-(2'-carboxymethylthioethyl)-cis-bicyclo[3,3,0]octane

2β-(3'-hydroxy-3'-methyl-trans-1'-octenyl)-3α-hydroxy-7-(2'-carboxymethylthioethyl)-cis-bicyclo[3,3,0]-octane

2β-(3'-hydroxy-2'-methyl-trans-1'-octenyl)-3α-hydroxy-7-(2'-carboxymethylthioethyl)-cis-bicyclo[3,3,0]-octane

2β-(3'-hydroxy-trans-1'-octenyl)-7-(2'-carboxymethylthioethyl)-cis-bicyclo[3,3,0]octane

2β-(3'-hydroxy-trans-1'-octenyl)-7-(2'-carboxymethylthioethyl)-cis-bicyclo[3,3,0]octane

2β-(3'-hydroxyoctyl)-3α-hydroxy-7-(2'-carboxymethylthioethyl)-cis-bicyclo[3,3,0]octane

2β-(3'-acetoxy-trans-1'-octenyl)-3α-acetoxy-7-(2'-carboxymethylthioethyl)-cis-bicyclo[3,3,0]octane

2β-(3'-benzoyloxy-trans-1'-octenyl)-3α-benzoyloxy-7-(2'-carboxymethylthioethyl)-cis-bicyclo[3,3,0]octane

2β-(3'-(tetrahydropyran-2"-yloxy)-trans-1'-octenyl)-3α-(tetrahydropyran-2"-yloxy)-7-(2'-carboxymethylthioethyl)-cis-bicyclo[3,3,0]octane

2β-(3'-(ethoxyethyl-1"-yloxy)-trans-1'-octenyl-3α-(ethoxyethyl-1"-yloxy)-7-(2'-carboxymethylthioethyl)-cis-bicyclo[3,3,0]octane

2β-(3'-hydroxy-trans-1'-octenyl)-3α-hydroxy-7-(2'-methoxycarbonylmethylthioethyl)-cis-bicyclo[3,3,0]-ontene

2β-(3'-hydroxy-trans-1'-octenyl)-3α-hydroxy-7-(2'-ethoxycarbonylmethylthioethyl)-cis-bicyclo[3,3,0]-octane

2β-(3'-hydroxy-trans-1'-octenyl)-3α-hydroxy-7-(2'-phenoxycarbonylmethylthioethyl)-cis-bicyclo[3,3,0]-octane

2β-(3'-hydroxy-trans-1'-octenyl)-3α-hydroxy-7-(2'-benzyloxycarbonylmethylthioethyl)-cis-bicyclo[3,3,0]-octane

2β-(3'-hydroxy-trans-1'-octenyl)-3α-hydroxy-7-(2'-methoxymethoxycarbonylmethylthioethyl)-cis-bicyclo-[3,3,0]octane

2β-(3'-hydroxy-trans-1'-octenyl)-3α-hydroxy-7-(2'-acetyloxymethoxycarbonylmethylthioethyl)-cis-bicyclo[3,3,0]octane

2β-(3'-hydroxy-trans-1'-octenyl)-3α-hydroxy-7-(2'-cyclopentyloxycarbonylmethylthioethyl)-cis-bicyclo-[3,3,0]octane

2β-(3'-hydroxy-trans-1'-octenyl)-3α-hydroxy-7-(2'-benzoylmethoxycarbonylmethylthioethyl)-cis-bicyclo-[3,3,0]octane

2β-(3'-hydroxy-trans-1'-octenyl)-3α-hydroxy-7-(2'-(2"-hydroxyethoxycarbonylmethylthioethyl)-cis-bicyclo[3,3,0]octane

2β-(3'-hydroxy-trans-1'-octenyl)-3α-hydroxy-7-(2'-aminocarbonylmethylthioethyl)-cis-bicyclo[3,3,0]-octane

2β-(3'-hydroxy-trans-1'-octenyl)-3α-hydroxy-7-(2'-(N,N-dimethylaminocarbonylmethylthio)ethyl)-cis-bicyclo[3,3,0]octane

2β-(3'-hydroxy-trans-1'-octenyl)-3α-hydroxy-7-(2'-(N-cyclohexylaminocarbonylmethylthio)ethyl)-cis-bicyclo[3,3,0]octane

2β-(3'-hydroxy-trans-1'-octenyl)-3α-hydroxy-7-(2'-(N-benzylaminocarbonylmethylthio)ethyl)-cis-bicyclo[3,3,0]octane

2β-(3'-hydroxy-trans-1'-octenyl)-3α-hydroxy-7-(2'-(N-benzylanilinocarbonylmethylthio)ethyl)-cis-bicyclo[3,3,0]octane

2β-(3'-hycroxy-trans-1'-octenyl)-3α-hydroxy-7-(2'-pyrrolidinocarbonylmethylthioethyl)-cis-bicyclo[3,3,0]-octane

2β-(3'-hydroxy-trans-1'-octenyl)-3α-hydroxy-7-(2'-anilinocarbonylmethylthioethyl)-cis-bicyclo[3,3,0]octane

2β-(3'-hydroxy-trans-1'-octenyl)-3α-hydroxy-7-(2'-morholinocarbonylmethylthioethyl)-cis-bicyclo[3,3,0]octane

Practical and preferred embodiments of the present invention are illustratively shown in the following example, which are not intended to limit the scope of the invention thereto.

Referential Example 1

A tetrahydrofuran solution (100 ml) of 8-oxo-cis-bicyclo[4,3,0]non-3-ene (80 g) was added to a tetrahydrofuran solution (1,000 ml) of the ylide prepared with triethyl phosphonoacetate (160 g) and sodium hydride (60% mineral oil dispersion, 23.5 g). The mixture was stirred for 30 min. at room temperature and poured into water and then extracted with ethylacetate. The organic layer was washed with water, dried, concentrated under reduced pressure and then chromatographed to give 8-ethoxycarbonylmethylene-cis-bicyclo[4,3,0]non-3-ene.

NMR δ ($CDCl_3$) 4.14 (2H, q, J=7 Hz), 5.61 (2H, br) 5.80 (1H, m)

IR $\nu_{cm^{-1}}^{film}$ 2900, 1705

Referential Example 2

To liquid ammonia (850 ml), was added a mixed solution of 8-ethoxycarbonylmethylene-cis-bicyclo[4,3,0]-non-3-ene (24.8 g), ethanol (180 ml) and ether (70 ml) at -50°C, and then the mixture was stirred for 10 min. Lithium (9.6 g) was added by portions, and then the mixture was stirred for 2 hr. at -50°C. Ammonia was evaporated and the mixture was poured into water, and then extracted with ethylacetate. The extract was washed with water, dried, concentrated and chromatographed to give 8-(2'-hydroxyethyl)-cis-bicyclo[4,3,0]-non-3-ene.

NMR δ ($CDCl_3$) 3.68 (2H, t, J=7Hz), 5.67 (2H, brds)

Referential Example 3

To an ethanol suspension (250 ml) of sodium borohydride (7.2 g), was added 8-oxo-cis-bicyclo[4,3,0]-non-3-ene (100 g), and then the mixture was stirred for 2 hr. at 5°C to 10°C. The mixture was poured into water, and extracted with ethylacetate. The organic layer was washed with water, dried, concentrated under reduced pressure to give 8-hydroxy-cis-bicyclo [4,3,0]non-3-ene.

NMR δ ($CCl_4$) 4.0-4.5 (1H, m), 5.65 (2H, m)

Referential Example 4

To a mixed solution of 8-hydroxy-cis-bicyclo-[4,3,0]non-3-ene (109 g), triethylamine (160 g) and

1 toluene (200 ml), was added methanesulfonyl chloride (110 g) at 0°C to 5°C. After stirred for one hr., the reaction mixture was poured into water and then extracted with ethylacetate. The organic layer was washed with water, dried, and concentrated to give 8-methanesulfonyloxy-cis-bicyclo[4,3,0]non-3-ene.

$IR^{film}_{cm^{-1}}$ 2930, 1665, 1430, 1350, 1180, 935, 885

Sodium (18 g) was added to ethanol (300 ml) by portions at room temperature. After the sodium dissolved, an ethanol solution (200 ml) of diethyl malonate (131 g) was added and the mixture was stirred for 30 min. An ethanol solution (200 ml) of 8-methanesulfonyloxy-cis-bicyclo[4,3,0]non-3-ene (160 g) obtained above was added at room temperature and then the mixture was stirred under reflux for 7 hr. The mixture was cooled and poured into water, and then extracted with toluene. The organic layer was dried, concentrated, and distilled in vacuo to give 8-(diethoxycarbonyl)methyl-cis-bicyclo-[4,3,0]non-3-ene.

bp. 125-140°C/0.2 mmHg

Referential Example 5

To a mixed solution of 8-(diethoxycarbonyl)-methyl-cis-bicyclo[4,3,0]non-3-ene (120 g), dimethylsulfoxide (600 ml), water (12 g), was added sodium chloride (19 g), and then the mixture was stirred under reflux for 7 hr. The mixture was poured into water, and extracted with n-hexane. The organic layer was

dried, concentrated, and then distilled in vacuo to give 8-ethoxycarbonylmethyl-cis-bicyclo[4,3,0]non-3-ene.

bp. 107-125°C/0.2 mmHg

Referential Example 6

To a tetrahydrofuran suspension (250 ml) of lithium aluminum hydride (1.14 g), was added a tetrahydrofuran solution of 8-ethoxycarbonylmethyl-cis-bicyclo[4,3,0]non-3-ene (10.4 g) and the mixture was stirred under reflux for 3 hr. The mixture was cooled to 5-10°C, and treated by successive dropwise addition of water (1 ml), 15% sodium hydroxide solution (1 ml), water (3 ml). A dry granular precipitate was filtered out and the mother liquor was concentrated to give 8-(2'-hydroxyethyl)-cis-bicyclo[4,3,0]non-3-ene.

Referential Example 7

To a mixed solution of 8-(2'-hydroxyethyl)-cis-bicyclo[4,3,0]non-3-ene (10 g), triethylamine (9.1 g) and tetrahydrofuran (50 ml), was added methanesulfonyl chloride (10.3 g) at 0°C to 5°C. After stirred for one hr., the reaction mixture was poured into water and then extracted with ethylacetate. The organio layer was washed with water, dried and concentrated to give 8-(2'-methanesulfonyloxyethyl)-cis-bicyclo[4,3,0]non-3-ene.

To a suspension of ethyl thioglycolate (10.8 g), sodium hydride (4.3 g, 60% mineral oil dispersion), and

ethanol (100 ml), was added 8-(2'-methanesulfonyloxy--ethyl)-cis-bicyclo[4,3,0]non-3-ene obtained above and the mixture was stirred under reflux for 2 hr. After the mixture was cooled to room temperature, ethylacetate (5.4 g) was added, and then the mixture was concentrated under reduced pressure. The residue was diluted with water, and extracted with hexane. The extract was dried and concentrated to give 8-(2'-ethoxycarbonylmethyl-thioethyl)-cis-bicyclo[4,3,0]non-3-ene.

NMR δ ($CDCl_3$) 1.27 (3H, t), 2.62 (2H, t), 3.20 (2H, S), 4.16 (2H, q), 5.57-5.7 (2H, m)

Referential Example 8

A mixed solution of 8-(2'-ethoxycarbonyl-methylthioethyl)-cis-bicyclo[4,3,0]non-3-ene (1.5 g), methanol (50 ml), and dichloromethane (20 ml) was subjected to a stream of ozonized oxygen at -50°C. After the starting material was disappeared, dimethylsulfide (12 ml) was added and the mixture was stirred for 2 hr. at -20°C to -5°C. The mixture was then concentrated by introduction of a stream of nitrogen to give an oily dialdehyde.

The dialdehyde thus obtained was dissolved in methanol (50 ml) and potassium carbonate (0.7 g) was added at 0°C. The mixture was stirred for one hr. and the precipitate was removed by filtration to give a methanol solution of 2β-formyl-3α-hydroxy-7-(2'-ethoxy-carbonylmethylsulfinylethyl)-cis-bicyclo[3,3,0]octane.

The methanol solution obtained above was added to tetrahydrofuran solution (250 ml) of the ylide prepared with dimethyl(2-oxo-4-methyloctyl)phosphonate (4.7 g) and sodium hydride (60%, 0.36 g). After the mixture was stirred for one hr. at room temperature, acetic acid (0.54 g) was added. The mixture was concentrated under reduced pressure and poured into water, and then extracted with ethylacetate. The extract was washed with water, dried and concentrated under reduced pressure and then chromatographed on silica gel to give 2β-(3'-oxo-5'methyl-trans-1'-nonenyl)-3α-hydroxy-7-(2'-ethoxycarbonylmethylsulfinylethyl)-cis-bicyclo[3,3,0]octane.

NMR δ ($CDCl_3$) 0.89-1.03 (6H, m), 3.80 (2H, S), 4.02 (2H, q), 6.10 (1H, d), 6.73 (1H, dd)

Referential Example 9

According to the same procedures as Referential Example 8, there was obtained the following compound.

2β-(3'-oxo-4'-(3"-trifluoromethylphenoxy)-trans-1'-butenyl)-3α-hydroxy-7-(2'-ethoxycarbonylmethylsulfinylethyl)-cis-bicyclo[3,3,0]octane.

NMR δ ($CDCl_3$) 1.31 (3H, t), 3.80 (2H, S), 4.10 (2H, q), 4.82 (2H, S), 6.65 (1H, d), 6.9-7.75 (5H, m)

Referential Example 10

A methanol solution (30 ml) of 8-ethoxycarbonylmethyl-cis-bicyclo[4,3,0]non-3-ene (2.08 g) was subjected to a stream of ozonized oxygen at -40°C. After the starting material was disappeared, dimethylsulfide (10 ml) was added and the mixture was stirred for one hr. at -5°C to 0°C. The mixture was then concentrated by introduction of a stream of nitrogen to give an oily dialdehyde.

The dialdehyde thus obtained was dissolved in methanol (20 ml) and an aqueous sodium hydroxide (5%, 10 ml) was added at -10°C to -5°C. The mixture was stirred for 30 min at the same temperature and then poured into a mixture of water and ethylacetate. After separation, the organic layer was washed with water, dried and concentrated under reduced pressure to give an oily 2β-formyl-3α-hydroxy-7-ethoxycarbonylmethyl-cis-bicyclo[3,3,0]octane.

A tetrahydrofuran solution (THF, 10 ml) of the aldol obtained above was added to THF solution (150 ml) of the ylide prepared with dimethyl(2-oxo-heptyl)-phosphonate (3.3 g) and sodium hydride (60%, 0.72 g). The mixture was stirred for one hr. at room temperature, and then poured into water, and extracted with ethylacetate. The extract was washed with water, dried and concentrated under reduced pressure and then chromatographed on silica gel to give 2β-(3'-oxo-trans-1'-octenyl)-3α-hydroxy-7-ethoxycarbonylmethyl-cis-bicyclo-[3,3,0]octane.

NMR δ ($CDCl_3$) 0.9 (3H, m), 3.57-3.93 (1H, m),

4.11 (2H, q), 6.15 (1H, d), 6.75 (1H, dd)

1 Referential Example 11

According to the same procedure as Referential Example 10, there were obtained the following compounds.

2β-(3'-oxo-trans-1'-nonenyl)-3α-hydroxy-7-ethoxycarbonylmethyl-cis-bicyclo[3,3,0]octane.

NMR δ ($CDCl_3$) 0.9 (3H, m), 6.15 (1H, d), 6.77 (1H, dd)

2β-(3'-oxo-4'-phenoxy-trans-1'-butenyl)-3α-hydroxy-7-ethoxycarbonylmethyl-cis-bicyclo[3,3,0]octane.

NMR δ ($CDCl_3$) 1.23 (3H, t), 4.08 (2H, q), 4.70 (2H, S), 6.45 (1H, d), 6.67-7.4 (6H, m)

Referential Example 12

To an ethanol solution (100 ml) of 2β-(3'-oxo-trans-1'-octenyl)-3α-hydroxy-7-ethoxycarbonylmethyl-cis-bicyclo[3,3,0]octane (1.66 g) was added an ethanol solution (100 ml) of sodium borohydride, and the mixture was stirred for one hr. at -30°C to -20°C. The mixture was poured into water and extracted with ethylacetate. The extract was washed with water, dried, concentrated at reduced pressure and chromatographed to give 2β-(3'β-hydroxy-trans-1'-octenyl)-3α-hydroxy-7-(ethoxycarbonylmethyl)-cis-bicyclo[3,3,0]octane.

NMR δ ($CDCl_3$) 0.9 (3H, m), 3.97-4.2 (1H, m), 4.13 (2H, q), 5.5-5.65 (2H, m)

and 2β-(3'α-hydroxy-trans-1'-octenyl)-3α-hydroxy-7-(ethoxycarbonylmethyl)-cis-bicyclo[3,3,0]octane.

NMR δ ($CDCl_3$) 0.89 (3H, m), 3.85-4.10 (1H, m), 4.07 (2H, q), 5.4-5.53 (2H, m)

1 Referential Example 13

According to the same procedure as Referential Example 12, there were obtained the following compounds.

2β-(3'β-hydroxy-trans-1'-nonenyl)-3α-hydroxy-7-ethoxycarbonylmethyl-cis-bicyclo[3,3,0]octane.

NMR δ ($CDCl_3$) 0.9 (3H, m), 3.93-4.17 (1H, m), 5.53-5.67 (2H, m)

2β-(3'α-hydroxy-trans-1'-nonenyl)-3α-hydroxy-7-ethoxycarbonylmethyl-cis-bicyclo[3,3,0]octane.

NMR δ ($CDCl_3$) 0.9 (3H, m), 5.47-5.60 (2H, m)

2β-(3'β-hydroxy-4'-phenoxy-trans-1'-butenyl)-3α-hydroxy-7-ethoxycarbonylmethyl-cis-bicyclo[3,3,0]-octane.

NMR δ ($CDCl_3$) 1.27 (3H, t), 3.93 (2H, m), 4.10 (2H, q), 4.33-4.6 (1H, m), 5.47-5.97 (2H, m), 6.8-7.1 (3H, m), 7.1-7.4 (2H, m)

2β-(3'α-hydroxy-4'-phenoxy-trans-1'-butenyl)-3α-hydroxy-7-ethoxycarbonylmethyl-cis-bicyclo[3,3,0]-octane.

NMR δ ($CDCl_3$) 1.27 (3H, t), 4.12 (2H, q), 4.33-4.6 (1H, m), 5.6-5.73 (2H, m), 6.8-7.03 (3H, m), 7.17-7.4 (2H, m)

Referential Example 14

To a mixed solution of 2β-(3'β-hydroxy-trans-1'-octenyl)-3α-hydroxy-7-ethoxycarbonyl-cis-bicyclo-[3,3,0]octane (0.3 g), 2,3-dihydropyran (0.34 g), and dichloromethane (20 ml), was added pyridinium p-toluenesulfonate (100 mg), and then the mixture was stirred for 5 hr. at room temperature. The mixture was poured into aqueous sodium bicarbonate and extracted with ethylacetate. The extract was washed with water, dried, and concentrated under reduced pressure to give 2β-(3'β-(tetrahydropyran-2"-yloxy)-trans-1'-octenyl)-3α-(tetrahydropyran-2'-yloxy)-7-ethoxycarbonylmethyl-cis-bicyclo[3,3,0]octane.

NMR δ ($CDCl_3$) 0.9 (3H, m), 4.10 (2H, q), 4.5-4.8 (2H, m), 5.1-5.7 (2H, m)

A THF solution (10 ml) of the oil obtained above was added to THF suspension (20 ml) of lithium aluminum hydride (0.2 g) at room temperature and the mixture was stirred at the same temperature for 30 min. After the mixture was stirred under reflux for 30 min., an aqueous sodium hydroxide (15%, 1 ml) was added. The granular precipitate was filtered out and the mother liquor was concentrated under reduced pressure to give 2β-(3'β-(tetrahydropyran-2"-yloxy)-trans-1'-octenyl)-3α-(tetrahydropyran-2'-yloxy)-7-(2'-hydroxyethyl)-cis-bicyclo[3,3,0]octane.

Referential Example 15

According to the same procedure as Referential Example 14, there were obtained the following compounds.

2β-(3'α-(tetrahydropyran-2"-yloxy)-trans-1'-octenyl)-3α-(tetrahydropyran-2'-yloxy)-7-ethoxycarbonylmethyl-cis-bicyclo[3,3,0]octane.

NMR δ ($CDCl_3$) 0.89 (3H, m), 4.07 (2H, q), 4.47-4.8 (2H, m), 5.0-5.6 (2H, m)

2β-(3'α-(tetrahydropyran-2"-yloxy)-trans-1'-octenyl)-3α-(tetrahydropyran-2'-yloxy)-7-(2'-hydroxyethyl)-cis-bicyclo[3,3,0]octane.

NMR δ ($CDCl_3$) 0.89 (3H, m), 4.5-5.03 (2H, m), 5.25-5.7 (2H, m)

2β-(3'β-tetrahydropyran-2"-yloxy)-trans-1'-nonenyl)-3α-(tetrahydropyran-2'-yloxy)-7-(ethoxycarbonylmethyl)-cis-bicyclo[3,3,0]octane.

NMR δ ($CDCl_3$) 0.9 (3H, m), 1.25 (3H, t), 4.05 (2H, q), 4.43-5.0 (2H, m), 5.2-5.57 (2H, m)

2β-(3'α-(tetrahydropyran-2"-yloxy)-trans-1'-nonenyl)-3α-(tetrahydropyran-2'-yloxy)-7-(ethoxycarbonylmethyl)-cis-bicyclo[3,3,0]octane.

NMR δ ($CDCl_3$) 0.9 (3H, m), 1.25 (3H, t), 4.07 (2H, q), 4.5-5.0 (2H, m), 5.2-5.6 (2H, m)

2β-(3'β-(tetrahydropyran-2"-yloxy)-4'-phenoxy-trans-1'-butenyl)-3α-(tetrahydropyran-2'-yloxy)-7-(ethoxycarbonylmethyl)-cis-bicyclo[3,3,0]octane.

NMR δ ($CDCl_3$) 1.23 (3H, t), 4.07 (2H, q), 5.23-5.8 (2H, m), 6.7-7.0 (3H, m), 7.07-7.3 (2H, m)

2β-(3'α-(tetrahydropyran-2"-yloxy)-4'-phenoxy-trans-1'-butenyl)-3α-(tetrahydropyran-2'-yloxy)-7-

ethoxycarbonylmethyl-cis-bicyclo[3,3,0]octane.

NMR δ ($CDCl_3$) 1.27 (3H, t), 4.12 (2H, q), 5.27-5.83 (2H, m), 6.77-7.0 (3H, m), 7.1-7.47 (2H, m)

2β-(3'β-(tetrahydropyran-2"-yloxy)-trans-1'-nonenyl)-3α-(tetrahydropyran-2'-yloxy)-7-(2'-hydroxyethyl)-cis-bicyclo[3,3,0]octane.

NMR δ ($CDCl_3$) 0.9 (3H, m), 4.47-5.0 (2H, m), 5.33-5.6 (2H, m)

2β-(3'α-(tetrahydropyran-2"-yloxy)-trans-1'-nonenyl)-3α-(tetrahydropyran-2'-yloxy)-7-(2'-hydroxyethyl)-cis-bicyclo[3,3,0]octane.

NMR δ ($CDCl_3$) 0.9 (3H, m), 4.47-5.0 (2H, m), 5.2-5.6 (2H, m)

2β-(3'β-(tetrahydropyran-2"-yloxy)-4'-phenoxy-trans-1'-butenyl)-3α-(tetrahydropyran-2'-yloxy)-7-(2'-hydroxyethyl)-cis-bicyclo[3,3,0]octane.

NMR δ ($CDCl_3$) 5.23-5.8 (2H, m), 6.77-7.0 (3H, m) 7.13-7.37 (2H, m)

2β-(3'α-(tetrahydropyran-2"-yloxy)-4'-phenoxy-trans-1'-butenyl)-3α-(tetrahydropyran-2'-yloxy)-7-(2'-hydroxyethyl)-cis-bicyclo[3,3,0]octane.

NMR δ ($CDCl_3$) 4.33-5.0 (4H, m), 5.3-5.9 (2H, m) 6.8-7.03 (3H, m), 7.15-7.4 (2H, m)

Example 1

To an ethanol solution (50 ml) of 2β-(3'-oxo-5'-methyl-trans-1'-nonenyl)-3α-hydroxy-7-(2'-ethoxycarbonylmethylsulfinylethyl)-cis-bicyclo[3,3,0]octane

(0.35 g), was added sodium borohydride (100 mg), and the mixture was stirred for one hr. at -30°C to -20°C. The mixture was poured into water and extracted with ethylacetate. The extract was washed with water, dried, and concentrated under reduced pressure to give 2β-(3'-hydroxy-5'-methyl-trans-1'-nonenyl)-3α-hydroxy-7-(2'-ethoxycarbonylmethyl-sulfinylethyl)-cis-bicyclo[3,3,0]-octane as an oil.

NMR δ ($CDCl_3$) 0.8-1.0 (6H, m), 1.27 (3H, t)
4.05 (2H, q), 5.3-5.7 (2H, m)

Example 2

According to the same procedure as Example 1, there was obtained the following compound.

2β-(3'-hydroxy-4'-(3"-trifluoromethylphenoxy)-trans-1'-butenyl)-3α-hydroxy-7-(2'-ethoxycarbonyl-methylsulfinylethyl)-cis-bicyclo[3,3,0]octane.

NMR δ ($CDCl_3$) 1.30 (3H, t), 4.07 (2H, q), 5.30-5.70 (2H, m), 7.0-7.47 (4H, m)

Example 3

A mixture of 2β-(3'-hydroxy-5'-methyl-trans-1'-nonenyl)-3α-hydroxy-7-(2'-ethoxycarbonylmethylsulfinyl-ethyl)-cis-bicyclo[3,3,0]octane (0.12 g), ethanol (3 ml), and aqueous sodium hydroxide (1N, 3 ml) was stirred at room temperature for 3 hr. The mixture was poured into water and extracted with diethyl ether. The aqueous layer was acidified with aqueous solution of potassium

hydrogen sulfate and extracted with ethylacetate. The extract was washed with water, dried and concentrated under reduced pressure to give 2β-(3'-hydroxy-5'-methyl-trans-1'-nonenyl)-3α-hydroxy-7-(2'-carboxymethylsulfinylethyl)-cis-bicyclo[3,3,0]octane as an oil.

NMR δ ($CDCl_3$) 0.8-1.0 (6H, m), 4.0-4.3 (4H, m), 5.3-5.7 (2H, m)

Example 4

According to the same procedure as Example 3, there was obtained following compound.

2β-(3'-hydroxy-4'-(3"-trifluoromethylphenoxy)-trans-1'-butenyl)-3α-hydroxy-7-(2'-carboxymethyl-sulfinyl-ethyl)-cis-bicyclo[3,3,0]octane.

NMR δ ($CDCl_3$) 5.3-5.7 (2H, m), 6.9-7.5 (4H, m)

Example 5

A mixture of 2β-(3'-hydroxy-5'-methyl-trans-1'-nonenyl)-3α-hydroxy-7-(2'-ethoxycarbonylmethylsulfinyl-ethyl)-cis-bicyclo[3,3,0]octane (200 mg), 5% palladium on charcoal (100 mg) and ethanol (20 ml) was stirred under an atmosphere of hydrogen at room temperature for one hr. The mixture was filtered and washed with ethanol. The filtrate was condensed under reduced pressure to give 2β-(3'-hydroxy-5'-methylnonyl)-3α-hydroxy-7-(2'-ethoxy-carbonylmethylsulfinylethyl)-cis-bicyclo[3,3,0]-octane.

Example 6

According to the same procedure as Example 5 and Example 3, there were obtained the following compounds.

2β-(3'-hydroxy-4'-(3"-trifluoromethylphenoxy)-butyl)-3α-hydroxy-7-(2'-ethoxycarbonylmethylsulfinyl-ethyl)-cis-bicyclo[3,3,0]octane.

NMR δ ($CDCl_3$) 0.8-1.1 (6H, m), 7.0-7.47 (4H, m)

2β-(3'-hydroxy-5'-methylnonyl)-3α-hydroxy-7-(2'-carboxymethylsulfinylethyl)-cis-bicyclo[3,3,0]-octane.

NMR δ ($CDCl_3$) 0.8-1.1 (6H, m), 4.0-4.3 (4H, m)

2β-(3'-hydroxy-4'-(3"-trifluorophenoxy)butyl)-3α-hydroxy-7-(2'-carboxymethylsulfinylethyl)-cis-bicyclo-[3,3,0]octane.

NMR δ ($CDCl_3$) 6.9-7.5 (4H, m)

Example 7

To a mixture of 2β-(3'-hydroxy-5'-methylnonyl)-3α-hydroxy-7-(2'-carboxymethylsulfinylethyl)-cis-bicyclo-[3,3,0]octane (130 mg), ethanol (5 ml) and cobalt chloride (200 mg), was added sodium borohydride (160 mg) by portions at 0°C, and the mixture was stirred for 15 min. at same temperature. After the mixture was stirred for 2 hr. at room temperature, water was added. The mixture was acidified with aqueous solution of patassium hydrogen sulfate and then extracted with ethylacetate. The extract was washed with water, dried

and concentrated under reduced pressure to give 2β-(3'-hydroxy-5'-methylnonyl)3α-hydroxy-7-(2'-carboxymethylthioethyl)-cis-bicyclo[3,3,0]octane.

NMR δ ($CDCl_3$) 0.89-1.03 (6H, m)

Example 8

According to the same procedure as Example 7, there was obtained the following compound.

2β-(3'-hydroxy-4'-(3"-trifluoromethylphenoxy)-butyl)-3α-hydroxy-7-(2'-carboxymethylthioethyl)-cis-bicyclo[3,3,0]octane.

NMR δ ($CDCl_3$) 7.0-7.5 (4H, m)

Example 9

To a mixed solution of 2β-(3'β-(tetrahydropyran-2'-yloxy)-trans-1'-octenyl)3α-(tetrahydropyran-2'-yloxy)-7-(2'-hydroxyethyl)-cis-bicyclo[3,3,0]octane (0.1 g), triethylamine (50 mg) and tetrahydrofuran (5 ml), was added methanesulfonyl chloride (40 mg) at 0°C to 5°C, and the mixture was stirred for one hr. at the same temperature. The mixture was poured into water and extracted with ethylacetate. The extract was dried and concentrated to give 2β-(3'β-(tetrahydropyran-2'-yloxy)-trans-1'-octenyl)-3α-(tetrahydropyran-2'-yloxy)-7-(2'-methanesulfonyloxyethyl)-cis-bicyclo[3,3,0]octane as an oil.

A ethanol solution of the oil obtained above

was added to a solution of ethyl thioglycolate, sodium hydride (60%, 80 mg), and ethanol (10 ml) at room temperature, and then the mixture was refluxed for 2 hr. After the mixture was cooled to room temperature, acetic acid (0.12 g) was added. The mixture was poured into water and extracted with hexane. The extract was dried, concentrated and chromatographed to give 2β-(3'β-(tetrahydropyran-2"-yloxy)-trans-1'-octenyl)-3α-(tetrahydropyran-2'-yloxy)-7-(2'-ethoxycarbonylmethyl-thioethyl)-cis-bicyclo[3,3,0]octane.

NMR δ ($CDCl_3$) 0.9 (3H, m), 1.27 (3H, t), 3.20 (2H, S) 4.13 (2H, q), 4.53-4.97 (2H, m), 5.2-5.7 (2H, m)

Example 10

According to the same procedure as Example 9, there were obtained the following compounds.

2β-(3'α-(tetrahydropyran-2"-yloxy)-trans-1'-octenyl)-3α-(tetrahydropyran-2'-yloxy)-7-(2'-ethoxycarbonylmethylthioethyl)-cis-bicyclo[3,3,0]octane.

NMR δ ($CDCl_3$) 0.89 (3H, m), 1.30 (3H, t), 3.20 (2H, S), 4.15 (2H, q), 4.57-4.8 (2H, m), 5.23-5.67 (2H, m)

2β-(3'β-(tetrahydropyran-2"-yloxy)-trans-1'-nonenyl)-3α-(tetrahydropyran-2"-yloxy)-7-(2'-ethory-carbonylmethoxythioethyl)-cis-bicyclo[3,3,0]octane.

NMR δ ($CDCl_3$) 0.89 (3H, m), 2.5-2.7 (2H, t), 3.10 (2H, S), 4.5-4.67 (2H, m), 5.37-5.53 (2H, m)

2β-(3'α-(tetrahydropyran-2'-yloxy)-trans-1'-nonenyl)-3α-(tetrahydropyran-2'-yloxy)-7-(2'-ethoxycarbonylmethylthioethyl)-cis-bicyclo[3,3,0]octane.

NMR δ ($CDCl_3$) 0.9 (3H, m), 2.5-2.7 (2H, t), 3.07 (2H, S), 4.43-4.7 (2H, m), 5.3-5.53 (2H, m)

2β-(3'β-(tetrahydropyran-2"-yloxy)-4'-phenoxy-trans-1'-butenyl)-3α-(tetrahydropyran-2'-yloxy)-7-(2'-ethoxycarbonylmethylthioethyl)-cis-bicyclo[3,3,0]octane.

NMR δ ($CDCl_3$) 2.5-2.75 (2H, t), 3.10 (2H, S), 4.4-4.77 (2H, m), 5.4-5.7 (2H, m), 6.7-6.9 (3H, m), 7.07-7.2 (2H, m)

2β-(3'α-(tetrahydropyran-2"-yloxy)-4'-phenoxy-trans-1'-butenyl)-3α-(tetrahydropyran-2'-yloxy)-7-(2'-ethoxycarbonylmethylthioethyl)-cis-bicyclo[3,3,0]octane.

NMR δ ($CDCl_3$) 2.60 (2H, t), 3.07 (2H, S), 4.15 (2H, q), 5.33-5.8 (2H, m), 6.7-6.9 (3H, m), 7.07-7.3 (2H, m)

Example 11

To an ethanol solution (20 ml) of 2β-(3'β-(tetrahydropyran-2"-yloxy)-trans-1'-octenyl)-3α-(tetrahydropyran-2'-yloxy)-7-(2'-ethoxycarbonylmethylthioethyl)-cis-bicyclo[3,3,0]octane (50 mg), was added pyridinium p-toluenesulfonate (20 mg), and then the mixture was stirred for 2 hr. at 50°C-60°C. The mixture was concentrated under reduced pressure and chromatographed on silica gel to give 2β-(3'β-hydroxy-trans-1'-octenyl)-3α-hydroxy-7-(2'-ethoxycarbonylmethylthioethyl)-

1 cis-bicyclo[3,3,0]octane.

NMR δ ($CDCl_3$) 0.9 (3H, m), 1.30 (3H, t), 2.65 (2H, t), 3.25 (2H, S), 4.25 (2H, q), 5.3-5.7 (2H, m)

Example 12

According to the same procedure as Example 11, there were obtained the following compounds.

2β-(3'α-hydroxy-trans-1'-octenyl)-3α-hydroxy-7-(2'-ethoxycarbonylmethylthioethyl)-cis-bicyclo[3,3,0]-octane.

NMR δ ($CDCl_3$) 0.89 (3H, m), 1.27 (3H, t), 2.65 (2H, t), 3.23 (2H, S), 4.23 (2H, q), 5.3-5.7 (2H, m)

2β-(3'β-hydroxy-trans-1'-nonenyl)-3α-hydroxy-7-(2'-ethoxycarbonylmethylthioethyl)-cis-bicyclo[3,3,0]-octane.

NMR δ ($CDCl_3$) 0.9 (3H, m), 1.30 (3H, t), 2.65 (2H, t) 3.23 (2H, S), 4.20 (2H, q), 5.53-5.63 (2H, m)

2β-(3'α-hydroxy-trans-1'-nonenyl)-3α-hydroxy-7-(2'-ethoxycarbonylmethylthioethyl)-cis-bicyclo[3,3,0]-octane.

NMR δ ($CDCl_3$) 0.9 (3H, m), 1.25 (3H, t), 2.68 (2H, t), 3.20 (3H, S), 4.15 (2H, q), 5.43-5.57 (2H, m)

2β-(3'β-hydroxy-4'-phenoxy-trans-1'-butenyl)-3α-hydroxy-7-(2'-ethoxycarbonylmethylthioethyl)-cis-bicyclo[3,3,0]octane.

NMR δ ($CDCl_3$) 1.30 (3H, t), 2.67 (2H, t), 3.23 (2H, S), 3.87-4.0 (2H, d), 5.50-5.97 (2H, m),

6.87-7.07 (3H, m), 7.2-7.43 (2H, m)

2β-(3'α-hydroxy-4'-phenoxy-trans-1'-butenyl)-3α-hydroxy-7-(2'-ethoxycarbonylmethylthioethyl)-cis-bicyclo[3,3,0]octane.

NMR δ ($CDCl_3$) 2.65 (2H, t), 3.20 (2H, S), 3.93 (2H, d), 4.17 (2H, q), 4.37-4.6 (1H, m), 5.6-5.73 (2H, m), 6.8-7.03 (3H, m), 7.17-7.4 (2H, m)

Example 13

According to the same procedure as Example 3, there were obtained the following compounds.

2β-(3'β-hydroxy-trans-1'-octenyl)-3α-hydroxy-7-(2'-carboxymethylthioethyl)-cis-bicyclo[3,3,0]octane.

NMR δ ($CDCl_3$) 0.9 (3H, m), 2.65 (2H, t), 3.23 (2H, S), 5.3-5.73 (2H, m)

2β-(3'α-hydroxy-trans-1'-octenyl)-3α-hydroxy-7-(2'-carboxymethylthioethyl)-cis-bicyclo[3,3,0]octane.

NMR δ ($CDCl_3$) 0.89 (3H, m), 2.65 (2H, t), 3.23 (2H, t), 5.0-5.3(3H, br), 5.3-5.67 (2H, m)

2β-(3'β-hydroxy-trans-1'-nonenyl)-3α-hydroxy-7-(2'-carboxymethylthioethyl)-cis-bicyclo[3,3,0]octane.

NMR δ ($CDCl_3$) 0.9 (3H, m), 2.65 (2H, t), 3.23 (2H, S), 5.53-5.63 (2H, m)

2β-(3'α-hydroxy-trans-1'-nonenyl)-3α-hydroxy-7-(2'-carboxymethylthioethyl)-cis-bicyclo[3,3,0]octane.

NMR δ ($CDCl_3$) 0.9 (3H, S), 3.23 (2H, S), 5.43-5.57 (2H, m)

2β-(3'β-hydroxy-4'-phenoxy-trans-1'-butenyl)-

3α-hydroxy-7-(2'-carboxymethylthioethyl)-cis-bicyclo-[3,3,0]octane.

NMR δ ($CDCl_3$) 3.23 (2H, S), 5.5-6.0 (2H, m), 6.87-7.07 (3H, m), 7.2-7.43 (2H, m)

2β-(3'α-hydroxy-4'-phenoxy-trans-1'-butenyl)-3α-hydroxy-7-(2'-carboxymethylthioethyl)-cis-bicyclo-[3,3,0]octane.

NMR δ ($CDCl_3$) 2.68 (2H, t), 3.23 (2H, S), 5.2-5.3 (3H, br), 5.63-5.8 (2H, m), 6.87-7.07 (3H, m), 7.2-7.43 (2H, m)

Example 14

(1) To an ethanol solution (5 ml) of 2β-(3'α-(tetrahydropyran-2"-yloxy)-trans-1'-octenyl)-3α-(tetrahydropyran-2'-yloxy)-7-(2'-ethoxycarbonylmethylthioethyl)-cis-bicyclo[3,3,0]octane, was added hydrogen peroxixe (35% solution in water, 2 ml), and then the mixture was stirred for 2 hr. at room temperature. The mixture was diluted with chloroform and washed with water. The organic phase was dried and concentrated to give 2β-(3'α-(tetrahydropyran-2"-yloxy)-trans-1'-octenyl)-3α-(tetrahydropyran-2'-yloxy)-7-(2'-ethoxycarbonylmethylsulfinylethyl)-cis-bicyclo[3,3,0]octane as an oil.

NMR δ ($CDCl_3$) 0.89 (3H, m), 1.30 (3H, t), 3.66 (2H, S), 4.23 (2H, q), 4.5-4.8 (2H, m), 5.1-5.73 (2H, m)

(2) According to the same procedure as Example 11, the oil obtained above was converted to 2β-(3'α-hydroxy-trans-1'-octenyl)-3α-hydroxy-7-(2'-ethoxycarbonylmethyl-sulfinylethyl)-cis-bicyclo[3,3,0]octane.

NMR δ ($CDCl_3$) 3.67 (2H, S), 4.23 (2H, q), 5.37-5.5 (2H, m)

(3) In the same manner as Example 3, the compound obtained above was hydrolyzed to 2β-(3'α-hydroxy-trans-1'-octenyl)-3α-hydroxy-7-(2'-carboxymethylsulfinylethyl)-cis-bicyclo[3,3,0]octane.

NMR δ ($CDCl_3$) 0.9 (3H, m), 4.77-5.1 (3H, br), 5.43-5.6 (2H, m)

Example 15

To an ethanol solution (3 ml) of 2β-(3'α-hydroxy-trans-1'-octenyl)-3α-hydroxy-7-(2'-ethoxycarbonyl-methylthioethyl)-cis-bicyclo[3,3,0]octane (70 mg), was added hydrogen peroxide (35% solution in water, 1 ml), and then the mixture was stirred for one hr. The mixture was poured into water and extracted with ethylacetate. The organic phase was washed with aqueous sodium thiosulfate and brain, dried, and concentrated to give 2β-(3'α-hydroxy-trans-1'-octenyl)-3α-hydroxy-7-(2'-ethoxycarbonyl-methylsulfinylethyl)-cis-bicyclo[3,3,0]octane. This compound was identical to the compound obtained in Example 14-(2).

Example 16

(1) To an ethanol solution (3 ml) of 2β-(3'α-hydroxy-4'-phenoxy-trans-1'-butenyl)-3α-hydroxy-7-(2'-

ethoxy-carbonylmethylthioethyl)-cis-bicyclo[3,3,0]octane (60 mg), was added aqueous sodium periodate (0.5 M solution, 0.2 ml), and then the mixture was stirred for 12 hr. at room temperature. The mixture was diluted with water and extracted with ethylacetate. The organic phase was washed with aqueous sodium thiosulfate, dried, and concentrated to give 2β-(3'α-hydroxy-4'-phenoxy-trans-1'-butenyl)-3α-hydroxy-7-(2'-ethoxycarbonylmethylsulfinylethyl)-cis-bicyclo[3,3,0]octane.

NMR δ ($CDCl_3$) 1.27 (3H, t), 3.65 (2H, S), 4.22 (2H, q), 5.6-5.73 (2H, m), 6.8-7.0 (3H, m), 7.1-7.3 (2H, m)

(2) According to the same procedure as Example 3, the compound obtained above was hydrolyzed to 2β-(3'α-hydroxy-4'-phenoxy-trans-1'-butenyl)-3α-hydroxy-7-(2'-carboxymethylsulfinylethyl)-cis-bicyclo[3,3,0]octane.

NMR δ ($CDCl_3$) 3.33 (2H, brs), 3.95 (2H, d), 5.63-5.8 (2H, m), 6.83-7.03 (3H, m), 7.17-7.4 (2H,m)

Example 17

(1) To a methanol solution (10 ml) of 2β-(3'α-(tetrahydropyran-2"-yloxy)-trans-1'-octenyl)-3α-(tetrahydropyran-2'-yloxy)-7-(2'ethoxycarbonylmethylthiomethyl)-cis-bicyclo[3,3,0]octane (60 mg), was added aqueous sodium periodate (0.5 M solution, 0.2 ml), and then the mixture was stirred for 12 hr. at room temperature. The mixture was diluted with water, and extracted with ethylacetate. The organic phase was washed with aqueous sodium

thiosulfate, dried, and concentrated to give 2β-(3'α-(tetrahydropyran-2"-yloxy)-trans-1'-octenyl)-3α-(tetrahydropyran-2'-yloxy)-7-(2'-ethoxycarbonylmethylsulfinylethyl)-cis-bicyclo[3,3,0]octane. This compound was identical with the compound obtained in Example 14-(1).

(2) To a dichloromethane solution (10 ml) of the sulfinyl compound obtained above, were added sodium acetate (20 mg) and 3-chloroperoxybenzoic acid (21 mg), and then the mixture was stirred for 12 hr. at room temperature. The mixture was diluted with chloroform and washed with aqueous sodium thiosulfate, aqueous sodium bicarbonate, and brain successively. The organic phase was dried and concentrated to give 2β-(3'α-(tetrahydropyran-2"-yloxy)-trans-1'-octenyl) 3α-(tetrahydropyran-2'-yloxy)-7-(2'-ethoxycarbonylmethylsulfonyl)-cis-bicyclo-[3,3,0]octane.

NMR δ ($CDCl_3$) 0.89 (3H, m), 1.30 (3H, t), 3.2-3.4 (2H, m), 3.93 (2H, S), 4.25 (2H, q), 5.1-5.77 (2H, m)

(3) In the same manner as Example 11, the sulfonyl compound was converted to 2β-(3'α-hydroxy-trans-1'-octenyl)-3α-hydroxy-7-(2'-ethoxycarbonylmethylsulfonylethyl)-cis-bicyclo[3,3,0]octane.

NMR δ ($CDCl_3$) 0.9 (3H, m), 1.30 (3H, t), 3.93 (2H,S), 4.25 (2H,q), 5.4-5.57 (2H, m)

(4) According to the same procedure as Example 3, the compound obtained above was hydrolyzed to give

2β-(3'α-hydroxy-trans-1'-octenyl)-3α-hydroxy-7-(2'-carboxymethylsulfonylethyl)-cis-bicyclo[3,3,0]octane.

NMR δ ($CDCl_3$) 0.9 (3H, m), 3.5-3.8 (1H, m), 4.03 (2H, S), 5.43-5.57 (2H, m)

Example 18

To a dichloromethane solution (10 ml) of 2β-(3'α-(tetrahydropyran-2"-yloxy)-trans-1'-octenyl)-3α-(tetrahydropyran-2'-yloxy)-7-(2'-ethoxycarbonylmethylthioethyl)-cis-bicyclo[3,3,0]octane (60 mg), were added sodium acetate (20 mg) and 3-chloroperoxybenzoic acid (21 mg), and then the mixture was stirred for 12 hr. at room temperature. The mixture was diluted with chloroform and washed with aqueous sodium thiosulfate, aqueous sodium bicarbonate, and brain. The organic phase was dried and concentrated to give 2β-(3'α-(tetrahydropyran-2"-yloxy)-trans-1'-octenyl)-3α-(tetrahydropyran-2'-yloxy)-7-(2'-ethoxycarbonylmethylsulfonylethyl)-cis-bicyclo[3,3,0]octane. This compound was identical with the compound obtained in Example 17-(2).

WHAT IS CLAIMED IS:

1. A compound of the formula:

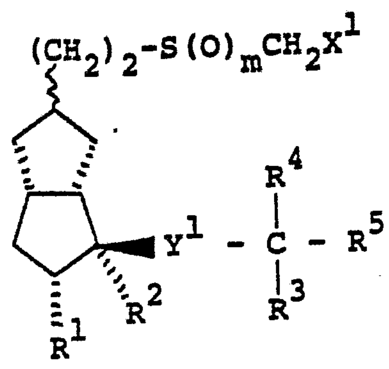

wherein $X^1$ is a free or esterified carboxyl group, or a group of the formula:

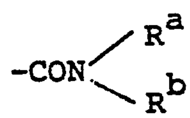

($R^a$ and $R^b$ are each independently a hydrogen atom, a $C_1$-$C_4$ alkyl group, a $C_3$-$C_7$ cycloalkyl group, a benzyl group, a phenyl group, a phenyl group substituted with a halogen atom or a $C_1$-$C_4$ alkyl group, or, when taken together with the adjacent nitrogen atom to which they are attached, they represent a 5 to 7 membered saturated heterocyclic group), $Y^1$ is a group of the formula:

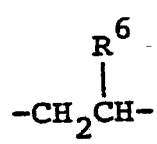

($R^6$ is a hydrogen atom or a $C_1$-$C_4$ alkyl group), or

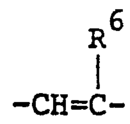

($R^6$ is as defined above),

$R^1$ is a hydrogen atom, a hydroxyl group or a protected hydroxyl group, $R^2$ is a hydrogen atom or $R^1$ and $R^2$, when taken together, mean a single linkage to form a double bond between the carbon atoms which they are linked, $R^3$ is a hydroxyl group or a protected hydroxyl group, $R^4$ is a hydrogen atom or a $C_1$-$C_4$ alkyl group, $R^5$ is a hydrogen atom, a $C_1$-$C_{12}$ alkyl group, a $C_2$-$C_{12}$ alkenyl group, a $C_2$-$C_{12}$ alkynyl group, a $C_3$-$C_{10}$ cycloalkyl group, a $C_4$-$C_{10}$ cycloalkenyl group, a hydroxy $C_1$-$C_{12}$ alkyl group, a $C_3$-$C_{10}$ heterocyclic group, a phenyl group optionally substituted with a halogen atom, a hydroxyl group, a $C_1$-$C_4$ alkyl group, a trifluoromethyl group, or a $C_1$-$C_4$ alkoxy group or a group of the formula: A-B (A is a $C_1$-$C_7$ alkylene chain and B is a $C_3$-$C_{10}$ cycloalkyl group, a $C_4$-$C_{10}$ cycloalkenyl group, a $C_1$-$C_{12}$ alkoxy group, a $C_3$-$C_{10}$ cycloalkoxy group, a $C_4$-$C_{10}$ cycloalkenyloxy group, a $C_3$-$C_{10}$ heterocyclic group, or a phenyl or phenoxy group optionally substituted with a halogen atom, a hydroxy group, a $C_1$-$C_4$ alkyl group, a trifluoromethyl group or a $C_1$-$C_4$ alkoxy group), and m is 0, 1 or 2; or a non-toxic pharmaceutically acceptable salt thereof.

2. The compound of Claim 1 which is represented by the formula:

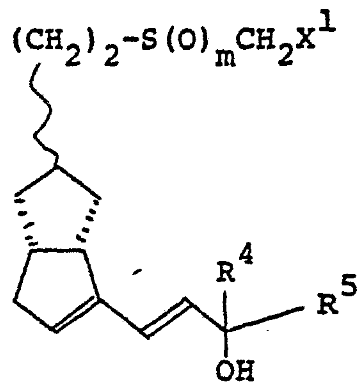

wherein $X^1$, $R^4$ and $R^5$ are each as defined in Claim 1.

3. The compound of Claim 1 which is represented by the formula:

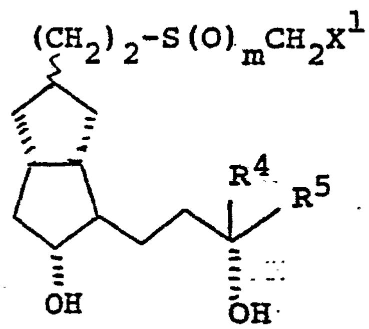

wherein $X^1$, $R^4$ and $R^5$ are each as defined in Claim 1.

4. The compound of Claim 1 which is represented by the formula:

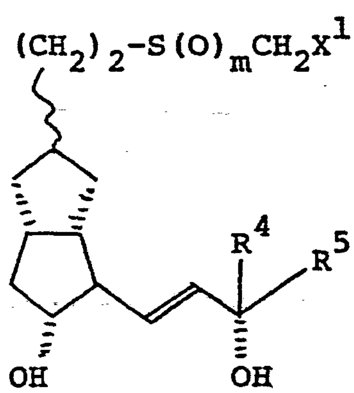

wherein $X^1$, $R^4$ and $R^5$ are each as defined in Claim 1.

5. The compound according to Claim 1, 2, 3, or 4 wherein the esterified carboxyl group is a $C_1$-$C_4$ alkoxycarbonyl group, an aryloxycarbonyl group, an aralkyloxycarbonyl group, a ($C_1$-$C_4$ alkoxy)methoxycarbonyl group, a ($C_2$-$C_5$ alkanoyloxy)carbonyl group, a ($C_3$-$C_7$ cycloalkylox-)carbonyl group, an arylcarbonylmethoxycarbonyl group or a (hydroxy $C_1$-$C_4$ alkoxy)carbonyl group.

6. The compound according to Claim 1, 2, 3, or 4 wherein $X^1$ is a free carboxyl group, a $C_1$-$C_4$ alkoxycarbonyl group, or a group of the formula:

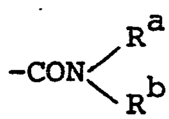

($R^a$ and $R^b$ are each independently a hydrogen atom, a $C_1$-$C_4$ alkyl group, a benzyl group or a phenyl group).

7. The compound according to Claim 4, wherein $X^1$ is a free carboxyl group or a $C_1$-$C_4$ alkoxycarbonyl group, $R^5$ is a $C_1$-$C_{12}$ alkyl group, a $C_2$-$C_{12}$ alkenyl group, a $C_2$-$C_{12}$ alkynyl group, a $C_3$-$C_{10}$ cycloalkyl group, or a group of the formula: $-CH_2-B$ (B is a phenoxy group optionally substituted with a halogen atom or a trifluoromethyl group).

8. The compound according to Claim 4, wherein $X^1$ is a free carboxyl group or a $C_1$-$C_4$ alkoxycarbonyl group, $R^4$ is a hydrogen atom and $R^5$ is a $C_1$-$C_{12}$ alkyl group, a phenoxymethyl group or a trifluoromethyl-

phenoxymethyl group.

9. 2β-(3'-Hydroxy-trans-1'-octenyl)3α-hydroxy-7-(2'-carboxymethylthioethyl)cis-bicyclo[3,3,0]octane or its stereoisomer.

10. 2β-(3'-Hydroxy-trans-1'-nonenyl)3α-hydroxy-7-(2'-carboxymethylthioethyl)cis-bicyclo[3,3,0]octane or its stereoisomer.

11. 2β-(3'-Hydroxy-5'-methyl-trans-1'-nonenyl)3α-hydroxy-7-(2'-carboxymethylthioethyl)cis-bicyclo[3,3,0]-octane or its stereoisomer.

12. 2β-(3'-Hydroxy-4'-phenoxy-trans-1'-butenyl)3α-hydroxy-7-(2'-carboxymethylthioethyl)cis-bicyclo[3,3,0]-octane or its stereoisomer.

13. 2β-[3'-Hydroxy-4'-(3"-trifluoromethylphenoxy)-trans-1'-butenyl]3α-hydroxy-7-(2'-carboxymethylthioethyl)cis-bicyclo[3,3,0]octane or its stereoisomer.

14. A process for producing a compound of the formula:

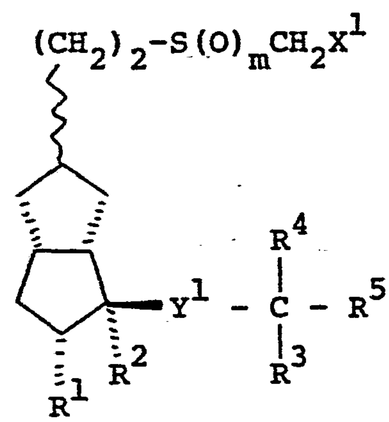

wherein $X^1$, $Y^1$, $R^1$, $R^2$, $R^3$, $R^4$ and $R^5$ are each as defined in Claim 1, or its nontoxic pharmaceutically

acceptable salt, which comprises reacting a compound of the formula:

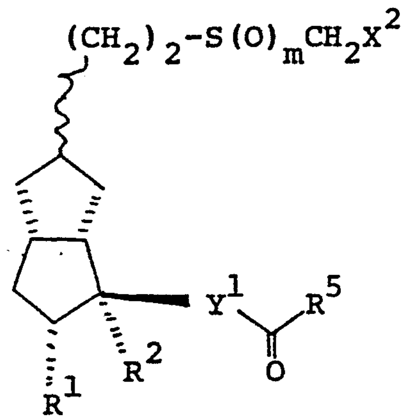

wherein $R^1$, $R^2$, $Y^1$ and $R^5$ are each as defined above, and $X^2$ is a free or esterified carboxyl group, a cyano group or a group of the formula:

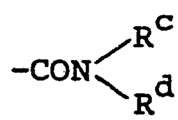

($R^c$ and $R^d$ are each independently a $C_1$-$C_4$ alkyl group, a $C_3$-$C_7$ cycloalkyl group, a benzyl group, a phenyl group, a phenyl group substituted with a $C_1$-$C_4$ alkyl group, or when taken together with the adjacent nitrogen atom to which they are attached, they represent a 5 to 7 membered saturated heterocyclic group), with a reducing agent or an organometalic compound of the formula:

$$M-R^7$$

wherein $R^7$ is a $C_1$-$C_4$ alkyl group and M is a lithium atom or a group of the formula: -Mg halo (halo is a halogen atom), optionally followed by oxidation of a sulfoxide

group, reduction of a sulfoxide group, hydrolysis of an amide group, an ester group or a cyano group, esterification of a carboxyl group, amidation of a free or esterified carboxyl group, reduction of a vinylene group, dehydrohalogenation of a halogenated vinylene group, protection of a hydroxy group and/or deprotection of a protected hydroxyl group.

15. The process according to Claim 14 wherein $X^2$ is a esterified carboxyl group or a group of the formula:

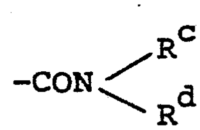

($R^c$ and $R^d$ are each as defined in Claim 14).

16. A process for producing a compound of the formula:

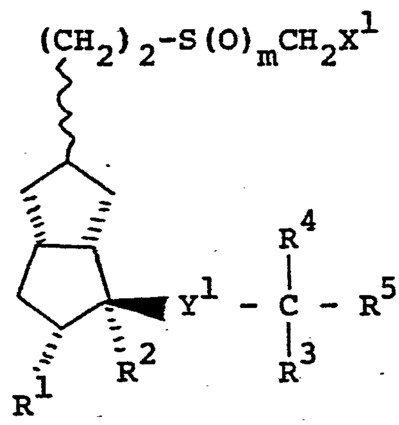

wherein $X^1$, $Y^1$, $R^1$, $R^2$, $R^3$, $R^4$ and $R^5$ are each as defined in Claim 1, or its non-toxic pharmaceutically acceptable salt, which comprises reacting a compound of the formula:

 

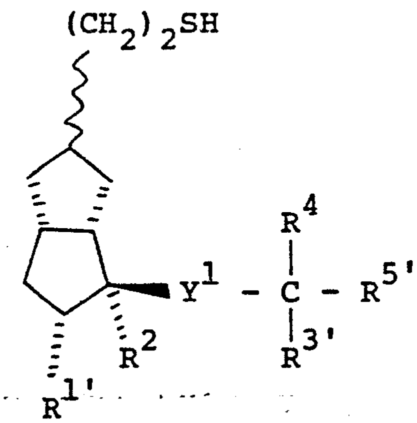

wherein $R^4$ and $Y^1$ are each as defined above and $R^{1'}$ is a hydrogen atom or a protected hydroxyl group, $R^2$ is a hydrogen atom, or $R^{1'}$ and $R^{2}$' when taken together, mean a single linkage to form a double bond between the carbon atoms to which they are linked, $R^{3'}$ is a protected hydroxy group and $R^{5'}$ is a hydrogen atom, a $C_1$-$C_{12}$ alkyl group, a $C_2$-$C_{12}$ alkenyl group, a $C_3$-$C_{12}$ alkynyl group, a $C_3$-$C_{10}$ cycloalkyl group, a $C_4$-$C_{10}$ cycloalkenyl group, a protected hydroxy $C_1$-$C_{12}$ alkyl group, a $C_3$-$C_{10}$ heterocyclic group, a phenyl group optionally substituted with a halogen atom, a protected hydroxy group, a $C_1$-$C_4$ alkyl group, a trifluoromethyl group or a $C_1$-$C_4$ alkyl group or a group of the formula: A-B' (A is a $C_1$-$C_7$ alkylene chain and B' is a $C_3$-$C_{10}$ cycloalkyl group, a $C_4$-$C_{10}$ cycloalkenyl group, a $C_1$-$C_{12}$ alkoxy group, a $C_3$-$C_{10}$ cycloalkoxy group, a $C_4$-$C_{10}$ cycloalkenyloxy group, a $C_3$-$C_{10}$ heterocyclic group, or a phenyl or a phenoxy group optionally substituted with a halogen atom, a protected hydroxy group, a $C_1$-$C_4$ alkyl group, a trifluoromethyl group or a $C_1$-$C_4$ alkyl group), with halogeno compound of

the formula:

$$\text{halo} - CH_2 - X^2$$

wherein $X^2$ is as defined in Claim 14 and halo is a halogen atom, optionally followed by oxidation of a sulfide group, hydrolysis of an amide group, a cyano group or an ester group, esterification of carboxyl group, amidation of a free or esterified carboxyl group, reduction of vinylene group, deprotection of a protected hydroxyl group and/or protection of a hydroxyl group.

17. A process for producing a compound of the formula:

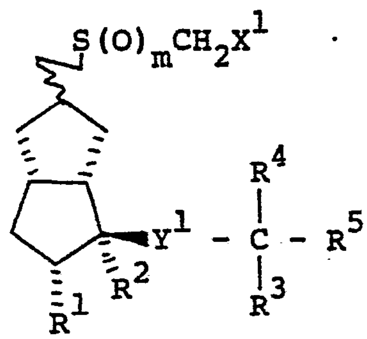

wherein $R^1$, $R^2$, $R^3$, $R^4$, $R^5$, m, $Y^1$ and $X^1$ are each as defined in Claim 1, or its non-toxic pharmaceutically acceptable salt, which comprises reacting a compound of the formula:

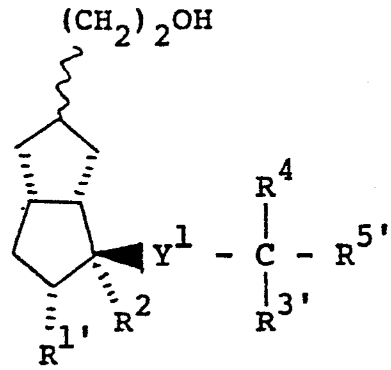

wherein $R^{1'}$, $R^{3'}$ and $R^{5'}$ are each as defined in Claim 16, and $R^2$, $R^4$ and $Y^1$ are each as defined above, with an alkylsulfonyl halide, an arylsulfonyl halide or a halogenating agent, and reacting of the resulting compound of the formula:

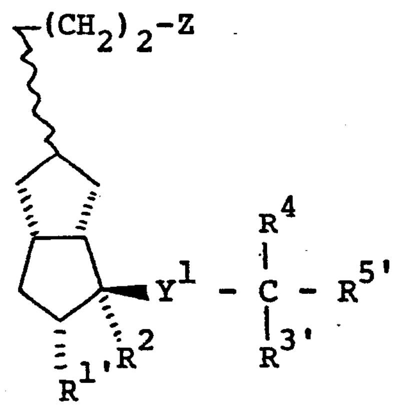

wherein $R^{1'}$, $R^2$, $R^{3'}$, $R^4$, $R^{5'}$ and $Y^1$ are each as defined above, and Z is a halogen atom, $C_1$-$C_4$ alkylsulfonyloxy group, an arylsulfonyloxy group, with the thiol compound of the formula:

$$HSCH_2X^2$$

wherein $X^2$ is as defined in Claim 14, optionally followed

by oxidation of a sulfide group, hydrolysis of an amide group, a cyano group or an ester group, esterification of carboxyl group, amidation of a free or esterified carboxyl group, reduction of vinylene group, deprotection of a protected hydroxyl group and/or protection of a hydroxyl group.

18. A compound of the formula:

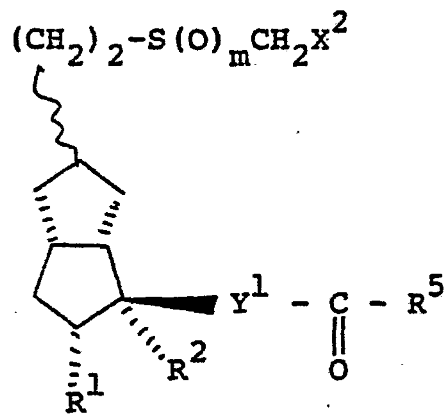

wherein $X^2$, $Y^1$, $R^1$, $R^2$ and $R^5$ are each as defined in Claim 1.

19. A compound of the formula:

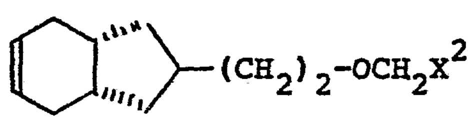

wherein $X^2$ is as defined in Claim 14.

20. A pharmaceutical composition which comprises a compound as claimed in Claim 1 and pharmaceutically acceptable carrier or diluent.

21. Use of a compound as claimed in Claim 1 as an antiulcer drug.